# FASCICULE DE BREVET EUROPEEN

(11) **EP 2 699 240 B1**
(45) Date de publication et mention de la délivrance du brevet: **27.05.2015**
(21) Numéro de dépôt: 12722749.4
(22) Date de dépôt: 17.04.2012
(51) Int. Cl.: A61K 31/4192, A61K 31/7056, A61P 35/02, C07H 19/056, C07D 249/06

(54) **DÉRIVÉS DE L'ACADÉSINE, PRODUITS ET COMPOSITIONS LES COMPRENANT, LEURS UTILISATIONS THÉRAPEUTIQUES ET LEURS PROCÉDÉS DE SYNTHÈSE**
ACADESIN-DERIVATE, PRODUKTE UND ZUSAMMENSETZUNGEN DAMIT, IHRE THERAPEUTISCHE VERWENDUNG UND VERFAHREN ZU IHRER SYNTHETISIERUNG
ACADESINE DERIVATIVES, PRODUCTS AND COMPOSITIONS INCLUDING SAME, THERAPEUTIC USES THEREOF, AND METHODS FOR SYNTHESIZING SAME

(30) Priorité: 18.04.2011 FR 1101204
(43) Date de publication de la demande: 26.02.2014
(73) Titulaire: Université Nice Sophia Antipolis, 06103 Nice Cedex 2 (FR); Centre National de la Recherche Scientifique, 75794 Paris Cedex 16 (FR); Institut National de la Santé et de la Recherche Médicale, 75013 Paris (FR)
(72) Inventeur: BENHIDA, Rachid, F-06000 Nice (FR); AUBERGER, Patrick, F-06100 Nice (FR); MALNUIT, Vincent, 18200 Prague 8 (CZ); DRIOWYA, Mohsine, F-59800 Lille (FR); PUISSANT, Alexandre, F-06100 Nice (FR); ROBERT, Guillaume, F-06100 Nice (FR)
(74) Mandataire: Agasse, Stéphane
(86) Numéro de dépôt international: PCT/FR2012/000147
(87) Numéro de publication internationale: WO 2012/143624

(56) Documents cités:
- WO-A1-2004/022572
- WO-A1-2009/060053
- VINCENT MALNUIT ET AL: "Tandem Azide-Alkyne 1,3-Dipolar Cycloaddition/Electrophilic Addition: A Concise Three-Component Route to 4,5-Disubstituted Triazolyl-Nucleosides", SYNLETT, vol. 2009, no. 13, 1 août 2009 (2009-08-01), pages 2123-2126, XP055012231, ISSN: 0936-5214, DOI: 10.1055/s-0029-1217560 cité dans la demande
- GUILLAUME ROBERT ET AL: "Acadesine Kills Chronic Myelogenous Leukemia (CML) Cells through PKC-Dependent Induction of Autophagic Cell Death", PLOS ONE, vol. 4, no. 11, 1 janvier 2009 (2009-01-01), page E7889, XP055012425, ISSN: 1932-6203, DOI: 10.1371/journal.pone.0007889 cité dans la demande

## Description

L'invention concerne des dérivés de l'acadésine, appelée aussi AICAR pour 5-Aminoimidazole-4-carboxamide-1-β-D-ribofuranoside, dans le domaine de la chimie du médicament. Plus particulièrement, l'invention concerne des dérivés de l'acadésine pour le traitement des cancers. Plus particulièrement encore, l'invention concerne des dérivés de l'acadésine pour le traitement des hémopathies myéloïdes telles que notamment la leucémie myéloïde chronique (LMC).

Les hémopathies malignes myéloïdes sont caractérisées par des altérations des propriétés de survie, de prolifération ou de différenciation des cellules des lignées myéloïdes. Elles sont provoquées par des anomalies génétiques généralement acquises affectant les cellules souches hématopoïétiques ou les progéniteurs (cellules souches différenciées ou « engagées ») engagés dans une voie particulière de différenciation. Ces hémopathies regroupent les leucémies aiguës myéloblastiques (LAM), les syndromes myélodysplasiques (SMD) et les syndromes myéloprolifératifs (SMP). La leucémie myéloïde chronique (LMC) est un syndrome myéloprolifératif (SMP) malin lié à une anomalie des cellules souches leucémiques conduisant à une production accrue de granulocytes à tous les stades de différenciation. Elle est causée par un défaut cytogénétique acquis résultant de la translocation réciproque de matériel génétique entre les bras long des chromosomes 9 et 22, la translocation (t9; 22) (q34-q11) ou chromosome Philadelphie. La conséquence moléculaire de ce réarrangement est la production d'une protéine chimérique p210BCR-ABL chez plus de 95% des patients atteints de leucémie myéloïde chronique (LMC).

BCR-ABL est une tyrosine kinase activée de façon constitutive qui explique l'insensibilité des cellules souches de patients à l'apoptose spontanée ou induite. Ces caractéristiques ont fait de BCR-ABL une cible pharmacologique privilégiée pour une intervention thérapeutique dans la leucémie myéloïde chronique (LMC).

Des progrès indéniables ont été enregistrés ces dernières années dans le traitement des cancers. Les prises en charge sont plus précoces et les méthodes de soin ont bénéficié de l'apport de nouveaux produits pharmaceutiques très efficaces contre les tumeurs. En quelques années, la greffe de moelle et surtout l'apparition de thérapies ciblées ont permis une meilleure prise en charge des patients. En effet, une des avancées significatives ces dernières années est l'arrivée sur le marché de l'imatinib mésylate (Glivec™).

Le Glivec™, formellement Mésylate d'Imatinib (IM), un inhibiteur pharmacologique de BCR-ABL, de c-KIT et du récepteur du PDGF est ainsi depuis une dizaine d'année le traitement de référence de la leucémie myéloïde chronique (LMC). Il est à ce jour le prototype des Inhibiteurs de Tyrosine Kinase (ITK) utilisés en thérapie anticancéreuse. Cependant, un pourcentage significatif de patients développe des résistances à ce composé et cette proportion augmente significativement lors des phases accélérées et aiguë de la maladie. Les mécanismes sous-tendant ces résistances sont variés et incluent des mutations ponctuelles dans le site de liaison de l'ATP de BCR-ABL dont la mutation T315I, qui génère des multirésistances et pose un véritable problème thérapeutique, des amplifications rares de BCR-ABL, des altérations des voies de signalisation situées en aval de cette kinase comme l'activation des SRC kinases.

Le Glivec™ permet à ce jour d'améliorer le pronostic de la maladie et d'obtenir des rémissions complètes dans environ 95% des cas. La chimiothérapie par l'Imatinib a ainsi remplacé progressivement la greffe comme traitement de première intention, les indications de greffe ont alors été réservées aux patients en échec sous Imatinib.

Toutefois, comme certains patients ne répondent pas à ce traitement ou développent des résistances, particulièrement lorsque la maladie est en phase de transformation aiguë, des stratégies alternatives à l'inhibition de BCR-ABL et visant à bloquer différentes voies de signalisation en aval ont été évaluées incluant l'inhibition des SRC kinases. Ces stratégies se sont révélées efficaces et des produits de substitution ont été évalués comme le nilotinib développé par la société Novartis™ ou le dasatinib (Sprycel™) développé par la société Brystol-Meyers Squibb™. Le Sprycel™ est actuellement prescrit aux patients résistant au Glivec™.

Cependant, certains patients, notamment ceux porteurs de la mutation T315I dans le site de liaison de l'ATP de p210BCR-ABL sont réfractaires à la quasi-totalité des Inhibiteurs de Tyrosine Kinase (ITK) utilisés dans le traitement de la leucémie myéloïde chronique (LMC). Il existe donc un véritable besoin de stratégies thérapeutiques alternatives, en particulier, pour tous les patients présentant la mutation T315I ou des résistances en aval de BCR-ABL.

L'acadésine ou 5-Aminolmidazole-Carboxamide Riboside (AICAR) est un nucléoside doté de très nombreux effets métaboliques, il est notamment décrit comme un activateur de l'AMP-activated protein Kinase (AMPK). Ce principe actif est actuellement en essai clinique de phase III pour la réduction des risques de complications cardio ou cérébrovasculaire au cours d'une chirurgie de pontage coronarien. Par ailleurs, une étude récente montre que ce composé induit l'apoptose de cellules B de leucémie lymphocytaire chronique (Campas et al, 2003), hémopathie pour laquelle il est en cours d'étude de phase I/II.

Il existe donc à ce jour un besoin de développer de nouveaux composés proposant une alternative aux produits existants ou en développement, afin de permettre notamment le traitement de patients résistants aux thérapies existantes, notamment aux patients résistants aux traitements de type Inhibiteurs de Tyrosine Kinase (ITK). Il existe également un besoin de développer de nouveaux composés permettant de répondre au problème de l'échappement thérapeutique. L'échappement thérapeutique ou tachyphylaxie désigne le ralentissement de l'effet thérapeutique après une période d'utilisation de médicaments. En outre, il existe aussi un besoin de développer des composés ayant des mécanismes d'action différents permettant le traitement de la leucémie myéloïde chronique et, de façon plus générale, le traitement de certains cancers. Enfin, il existe à ce jour un besoin de développer de nouveaux composés présentant une cytotoxicité inférieure aux composés actuellement disponibles.

La solution au problème posé a pour objet des composés de formule générale : 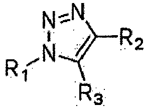 dans laquelle :
- R₁ est choisi parmi
   - un groupement pentose cyclique sous forme furanique avec les groupements OH libres ou éventuellement substitués par un ou plusieurs groupements mono-, bi- ou triphosphate (ou des prodrogues de ceux-ci), acétyle, isopropylidène, benzoyle ou para-toluoyle,
   - un groupement hexose sous forme pyranique avec les groupements OH libres ou éventuellement substitués par un ou plusieurs groupements mono-, bi- ou triphosphate (ou des prodrogues de ceux-ci) ou acétyle,
   - un groupement naphtyle, éventuellement substitué par un ou plusieurs groupements alkyles ou amines substituées ayant de 1 à 4 atomes de carbone,
   - un groupement benzyle éventuellement substitué par un ou plusieurs groupements alkyles ou amines substituées ayant de 1 à 4 atomes de carbone,
   - Des groupes phényles, biphényles, hétéroaryles ;
- R₂ est choisi parmi
   - un groupement amide -CONH₂, -CONHMe, -CONHEt, -CON(Me)₂, -CON(Et)₂,
   - un groupement acide ou ester -CO₂H, CO₂Me, CO₂Et, un groupement cyano ou amidine -CN, -C(NH₂)NH, -C(NHMe)NH, -C(NHEt)NH,
   - un groupement phényle éventuellement substitué par un halogène choisi parmi CI, Br, I et F,
   - un groupement thiophène,
   - un chaine carbonée linéaire ou ramifiée ayant de 3 à 10 atomes de carbone, ou
   - un groupement méthoxynaphtalène ; et
- R₃ est choisi parmi :
   - un groupement halogène,
   - un groupement furane ou -CO-furane,
   - un groupement thiophène ou -CO-thiophène ou -C≡C-thiophène,
   - un groupement toluoyle,
   - un groupement acétylène,
   - un groupement -CO-(CH₂)ₙ-CH₃, avec n compris entre 2 et 9,
   - un groupement phényle ou -C≡C-phényl, éventuellement substitué par un halogène,
   - un groupement -C≡C-CO₂Me, -C≡C-CO₂Et, -C≡C-CONH₂,
   - un groupement -C≡C-(CH₂)₆CH₃, ou
   - un groupement -C=C-2-méthoxynaphtalène ;
leurs racémiques, énantiomères, diastéréoisomères et leurs mélanges, leurs tautomères et leurs sels pharmaceutiquement acceptables, pour leur utilisation comme médicament.

De façon surprenante, le Demandeur a pu mettre en évidence que les composés selon l'invention permettent de diminuer la viabilité des lignées cellulaires leucémiques K562 sensibles à l'Imatinib et/ou des lignées cellulaires ImaR résistantes à l'Imatinib. Ceci est notamment démontré aux exemples 1 et 2.

En outre, le Demandeur a pu mettre en évidence que les composés selon l'invention ont une action sur différents types de mort cellulaire programmée (PCD).

La PCD joue un rôle fondamental dans le maintien du bon fonctionnement de tout organisme vivant car elle lui permet d'éliminer les cellules dont il n'a plus besoin.

Deux types majeurs de PCD sont connus : la PCD de type I appelée également apoptose et la PCD type II, appelée également autophagie.

Les caspases (protéases à cystéine) sont les acteurs principaux de l'apoptose. Elles clivent divers substrats protéiques essentiels à la survie cellulaire. Deux grandes voies conduisent à leur activation. La voie intrinsèque est orchestrée par les protéines de la famille Bcl-2 et déclenchée par le relargage de cytochrome C par la mitochondrie suivi de la formation d'un complexe multiprotéique (apoptosome) à l'origine de l'activation des caspases 9 puis 3. La voie extrinsèque est quant à elle déclenchée au niveau de la membrane plasmique par l'activation de récepteurs membranaires suivie de la formation d'un complexe multiprotéique (DISC) permettant l'activation des caspases 8 puis 3 (Grutter, 2000).

L'autophagie peut conduire soit à la survie, soit à la mort cellulaire. Son étape initiale est la formation de vacuoles à double membrane (les autophagosomes issus de la nucléation, l'élongation et la maturation d'un phagophore) qui piègent une portion de cytosol et les macromolécules et organites à éliminer. Les autophagosomes fusionnent avec les lysosomes pour former des autophagolysosomes, dans lesquels les hydrolases lysosomales (les cathepsines) dégradent leur contenu. La formation de l'autophagosome dépend du recrutement de protéines Atg et des protéines LC3-II et p62/SQSTM1 (Klionsky et al., 2008 ; Marino and Lopez-Otin, 2004). La voie mTOR intervient dans sa régulation. En effet, mTOR activée inhibe l'autophagie tandis que son inhibition par l'AMPK initie ce processus.

Comme cela est démontré dans l'exemple 1, les composés selon l'invention permettent d'agir sur les deux types de morts cellulaires programmées (PCD pour Programmed cell Death) exposées ci-dessus. Plus précisément, les mécanismes d'actions impliqués par les composés selon l'invention sont soit l'autophagie, soit l'apoptose soit une combinaison des deux types de ces mécanismes. Ceci est démontré dans l'exemple 1.

Le document intitulé « Acadesine Kills Chronic Myelogenous Leukemia (CML) Cells through PKC-Dependent Induction of Autophagic Cell Death » (Robert G et al., PloS ONE, November 2009, volume 4, Issue 11) décrit notamment que l'acadésine est efficace dans le traitement de la leucémie myéloïde chronique (LMC), et qu'elle agit sur la mort cellulaire programmée de type II, c'est-à-dire par un mécanisme d'autophagie. Toutefois, ce document ne divulgue pas de dérivés d'acadésine, ni leur activité éventuelle dans le traitement de la leucémie myéloïde chronique (LMC).

Le document intitulé « Tandem Azide-Alkyne 1,3-Dipolar Cycloaddition/Electrophilic Addition: A concise Three-Component Route to 4,5-Disubstituted Triazolyl-Nucleosides » (Malnuit V et al., Synlett 2009, No 13, pp 2123-2128) ne décrit pas les composés selon l'invention à titre de médicament. Ce document décrit la synthèse de dérivés de l'acadésine, ayant une formule générale différente des composés selon l'invention. De plus, même si ce document suggère que ces composés pourraient avoir des propriétés biologiques intéressantes, rien dans ce document ne suggère que ce type de composés puisse avoir une indication à titre de médicament et, plus particulièrement, dans le traitement du cancer comme par exemple la leucémie myéloïde chronique (LMC).

La présente invention a également pour deuxième objet un produit pharmaceutique contenant un composé selon l'invention et au moins un deuxième actif comme produit de combinaison pour une administration simultanée, séparée ou étalée dans le temps, pour son utilisation dans le traitement du cancer.

Elle a également pour troisième objet une composition pharmaceutique comprenant un composé selon l'invention et un support pharmaceutiquement acceptable.

L'invention sera mieux comprise à la lecture de la description non limitative qui suit, rédigée au regard des dessins annexés, dans lesquels :
- les figures 1 à 20 représentent les résultats d'études ayant pour but de cribler des composés selon l'invention en vue de déterminer leur efficacité et les mécanismes généraux d'actions de certains de ces composés.
- Les figures 21 à 23 présentent les résultats d'études de criblage et de dose-réponse de composés selon l'invention sur des lignées LMC (leucémie myéloïde chronique) sensibles (K562) à l'Imatinib.

Les composés selon l'invention sont des composés de formule générale : 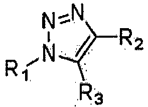 dans laquelle :
- R₁ est choisi parmi
   - un groupement pentose cyclique sous forme furanique avec les groupements OH libres ou éventuellement substitués par un ou plusieurs groupements mono-, bi- ou triphosphate (ou des prodrogues de ceux-ci), acétyle, isopropylidène, benzoyle ou para-toluoyle,
   - un groupement hexose sous forme pyranique avec les groupements OH libres ou éventuellement substitués par un ou plusieurs groupements mono-, bi- ou triphosphate (ou des prodrogues de ceux-ci) ou acétyle,
   - un groupement naphtyle, éventuellement substitué par un ou plusieurs groupements alkyles ou amines substituées ayant de 1 à 4 atomes de carbone,
   - un groupement benzyle éventuellement substitué par un ou plusieurs groupements alkyles ou amines substituées ayant de 1 à 4 atomes de carbone,
   - Des groupes phényles, biphényles, hétéroaryles ;
- R₂ est choisi parmi
   - un groupement amide -CONH₂, -CONHMe, -CONHEt, -CON(Me)₂, -CON(Et)₂,
   - un groupement acide ou ester -CO₂H, CO₂Me, CO₂Etun groupement cyano ou amidine -CN, -C(NH₂)NH, -C(NHMe)NH, -C(NHEt)NH,
   - un groupement phényle éventuellement substitué par un halogène choisi parmi CI, Br, I et F,
   - un groupement thiophène,
   - une chaine carbonée linéaire ou ramifiée ayant de 3 à 10 atomes de carbone, ou
   - un groupement méthoxynaphtalène ; et
- R₃ est choisi parmi :
   - un groupement halogène,
   - un groupement furane ou -CO-furane,
   - un groupement thiophène ou -CO-thiophène ou -C=C-thiophène,
   - un groupement toluoyle,
   - un groupement acétylène,
   - un groupement -CO-(CH₂)ₙ-CH₃, avec n compris entre 2 et 9,
   - un groupement phényle ou -C≡C-phényl, éventuellement substitué par un halogène,
   - un groupement -C≡C-CO₂Me, -C≡C-CO₂Et, -C≡C-CONH₂
   - un groupement -C≡C-(CH₂)₆CH₃, ou
   - un groupement -C=C-2-méthoxynaphtalène ;
leurs racémiques, énantiomères, diastéréoisomères et leurs mélanges, leurs tautomères et leurs sels pharmaceutiquement acceptables, pour leur utilisation comme médicament.

Les sels pharmaceutiquement acceptables sont des sels d'addition avec des acides inorganiques tels que chlorhydrate, bromhydrate, iodhydrate, sulfate, phosphate, diphosphate et nitrate ou avec des acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium.

De préférence, les composés selon l'invention sont des composés de formule générale : 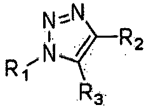 dans lesquels :
- R₁ est choisi parmi 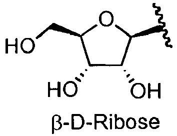 ou 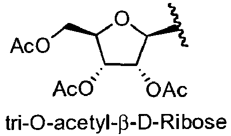 ou 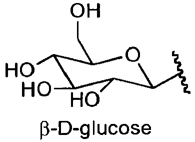 ou 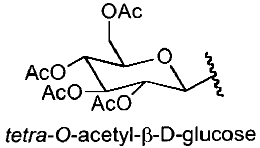 ou 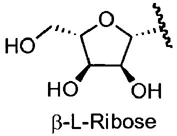 ou 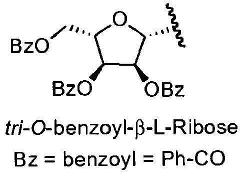 ou 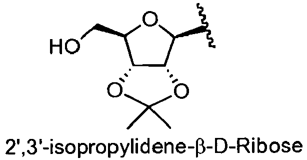 ou 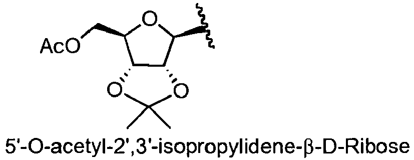 ou 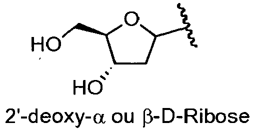 ou 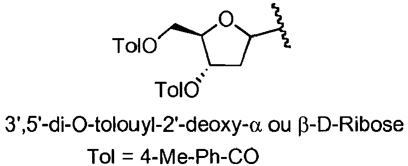 ou 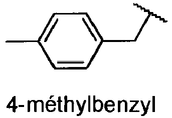 ou 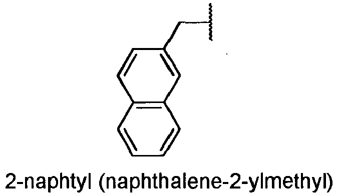
- R₂ est choisi parmi -CONH₂, CO₂Me, -CO₂Et, phényle, thiophène, -(CH₂)₆CH₃, p-fluoro-phényl ou le 2-méthoxynaphtalène ; et
- R₃ est choisi parmi I, CI, furane, CO-furane, CO-thiophène, acétylène, CO-(CH₂)₅-CH₃, toluoyle, -C≡C-CO₂Et, thiophène, phényl, -C≡C-phényl, -C≡C-thiophène,-C≡C-(CH₂)₆CH₃, -C≡C-(p-fluoro-phényl), ou -C≡C-2-méthoxynaphtalène ;
leurs racémiques, énantiomères, diastéréoisomères et leurs mélanges, leurs tautomères et leurs sels pharmaceutiquement acceptables, pour leur utilisation comme médicament.

De préférence encore, les composés selon l'invention sont des composés de formule générale : 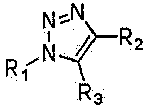 dans lesquels R₁ est 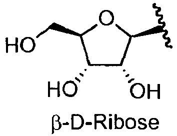 ou 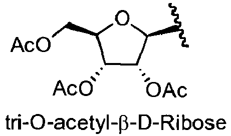 ou 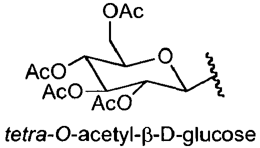 ou 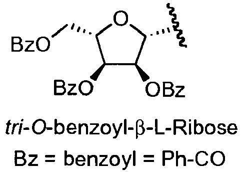 ou 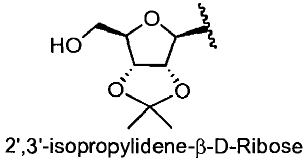 ou 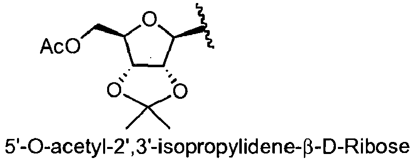 ou 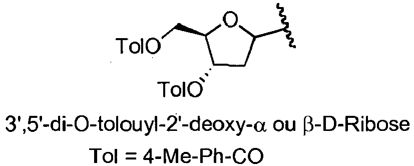 ou 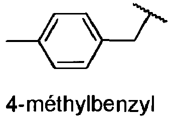 ou 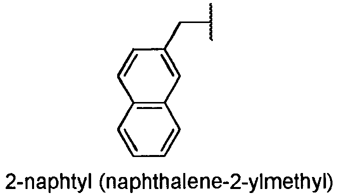 et
- lorsque R1 est un groupement β-D-ribose, alors :
   - R2= CONH₂ et R3 = I, CI, CO-Furane, CO-thiophène, toluoyle ou acétylène ;
      ou
   - R2= CO₂Me et R3 = I, furane ou acétylène ;
      ou
   - R2 = phényl et R3 = I ;
- lorsque R1 est un groupement tri-O-acétyl- β-D-ribose, alors :
   - R2= CO₂Et et R3 = CI, CO-(CH₂)₅-CH₃, CO-furane, toluoyle, -C≡C-CO₂Et, thiophène ou phényl ;
      ou
   - R2= phényl et R3 = -C≡C-phényl ;
      ou
   - R2 = thiophène et R3 = -C=C-thiophène ;
      ou
   - R2 = (CH₂)₆CH₃ et R3 = -C≡C-(CH₂)₆CH₃ ;
      ou
   - R2 = p-fluoro-phényl et R3 = -C≡C-p-fluoro-phényl ;
      ou
   - R2 = 2-methoxynaphtalène et
      R3 = -C=C-2-methoxynaphtalène ;
- lorsque R1 est un groupement tétra-O-acétyl- β-D-glucose, alors R2= CO₂Et et R3 = I ou -C≡C-CO₂Et ;
- lorsque R1 est un groupement tri-O-benzoyl-β-L-ribose, alors R2= CO₂Et et R3= -C≡C-CO₂Et ;
- lorsque R1 est un groupement 2',3'-isopropylidene-β-D-ribose, alors R2 = CO₂Et et R3= -C≡C-CO₂Et ou thiophène ;
- lorsque R1 = 5'-O-acetyl-2',3'-isopropylidene- β-D-ribose, alors R2 = CO₂Et et R3= -C≡C-CO₂Et ou thiophène ;
- lorsque R1 est un groupement 3',5'-di-O-tolouyl-2'-deoxy-β-D-ribose, alors R2= CO₂Et et R3= -C≡C-CO₂Et ;
- lorsque R1 est un groupement 4-méthylbenzyl, alors :
   - R2= CO₂Et et R3 = -C≡C-CO₂Et ;
      ou
   - R2= phényl et R3 = -C≡C-phényl ;
- lorsque R1 est un groupement 2-naphtyl (naphthalene-2-ylmethyl), alors :
   - R2= CO₂Et et R3 = I ;
      ou
   - R2= CO₂Et et R3 = -C≡C-CO₂Et ;
      ou
   - R2= Phényl et R3 = -C≡C-phényl ;
leurs racémiques, énantiomères, diastéréoisomères et leurs mélanges, leurs tautomères et leurs sels pharmaceutiquement acceptables, pour leur utilisation comme médicament.

De préférence encore, les composés selon l'invention sont des composés de formule générale : 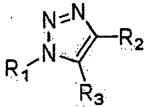 dans lesquels R1 est 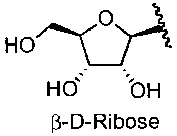 ou 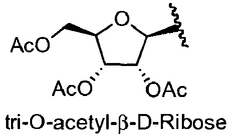 ou 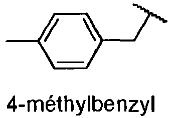 ou 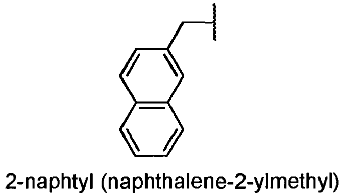 et
- lorsque R1 est un groupement β-D-ribose, alors :
   - R2= CONH₂ et R3 = CI, CO-furane, CO-thiophène ou toluoyle ;
      ou
   - R2= CO₂Me et R3 = I ou acétylène ;
      ou
   - R2 = phényl et R3 = I ;
- lorsque R1 est un groupement tri-O-acétyl- β-D-ribose, alors :
   - R2= CO₂Et et R3 = CO-(CH₂)₅-CH₃, CO-furane, toluoyle, -C≡C-CO₂Et, thiophène ou phényl ;
      ou
   - R2= phényl et R3 = -C=C-phényl ;
      ou
   - R2 = thiophène et R3 = -C≡C-thiophène ;
      ou
   - R2 = (CH₂)₆CH₃ et R3 = -C≡C-(CH₂)₆CH₃ ;
      ou
   - R2 = p-fluoro-phényl et R3 = -C=C-p-fluoro-phényl ;
      ou
   - R2 = 2-methoxynaphtalene et
      R3 = -C≡C-2-methoxynaphtalene ;
- lorsque R1 est un groupement 4-méthylbenzyl, alors :
   - R2= CO₂Et et R3 = -C≡C-CO₂Et ;
      ou
   - R2= phényl et R3 = -C≡C-phényl ;
- lorsque R1 est un groupement 2-naphtyl (naphthalene-2-yl-methyl), alors :
   - R2= CO₂Et et R3 = I ;
      ou
   - R2= CO₂Et et R3 = -C≡C-CO₂Et ;
      ou
   - R2= Phényl et R3 = -C≡C-phényl ;
leurs racémiques, énantiomères, diastéréoisomères et leurs mélanges, leurs tautomères et leurs sels pharmaceutiquement acceptables, pour leur utilisation comme médicament.

A titre d'exemples non limitatifs de composés préférés selon l'invention, on peut citer les composés :
- 1'-(4-ethoxycarbonyl-5-iodo-[1,2,3]-triazol-1-yl)-2',3',5'-tri-O-acétyl- β-D-ribofuranose ;
- 1'-(4-carbamoyl-5-iodo-[1,2,3]-triazol-1-yl)- β-D-ribofuranose;
- 1'-(4-méthoxycarbonyl-5-éthynyl-[1,2,3]-triazol-1-yl)- β-D-ribofuranose;
- 1-(naphtyl-2-methyl)-4-ethoxycarbonyl-5-iodo-1,2,3-triazole;
- 1-(naphtyl-2-methyl)-4-ethoxycarbonyl-5-éthylpropiolate-1,2,3-triazole;
- 1'-(4-ethoxycarbonyl-5-éthylpropiolate-[1,2,3]-triazol-1-yl)-2',3',5'-tri-O-acétyl- β-D-ribofuranose;
- 1'-(4-ethoxycarbonyl-5-(2-thienyl)-[1,2,3]-triazol-1-yl)-2',3',5'-tri-O-acétyl- β-D-ribofuranose;
- 1'-(4-ethoxycarbonyl-5-phényl-[1,2,3]-triazol-1-yl)-2',3',5'-tri-O-acétyl-- β-ribofuranose;
- 1-(4-méthylbenzyl)-4-ethoxycarbonyl-5-éthylpropiolate-1,2,3-triazole;
- 1'-(4-heptyl-5-(non-1-yn-1-yl)-[1,2,3]-triazol-1-yl)-2',3',5'-tri-O-acétyl- β-D-ribofuranose;
- 1'-(4-ethoxycarbonyl-5-éthylpropiolate-[1,2,3]-triazol-1-yl)-2',3',5'-tri-O-benzoyl- β-L-ribofuranose;
- 2'-désoxy-1'-(4-éthoxycarbonyl-5-éthylpropiolate-[1,2,3]-triazol-1-yl)-3',5'-di-O-(p-toluoyl)-α-D-ribofuranose;
- 1'-(4-ethoxycarbonyl-5-éthylpropiolate-[1,2,3]-triazol-1-yl)-2',3',4',6'-tétra-O-acétyl- β-D-glucopyranose;
- 1'-(4-ethoxycarbonyl-5-éthylpropiolate-[1,2,3]-triazol-1-yl)-2',3'-O-isopropylidène- β-D-ribofuranose;
- 1'-(4-ethoxycarbonyl-5-éthylpropiolate-[1,2,3]-triazol-1-yl)-2',3'-O-isopropylidène-5'-O-acétyl- β-D-ribofuranose;
- 1'-(4-ethoxycarbonyl-5-(2-thienyl)-[1,2,3]-triazol-1-yl)-2',3'-O-isopropylidène-β-D-ribofuranose; et
- 1'-(4-ethoxycarbonyl-5-(2-thienyl)-[1,2,3]-triazol-1-yl)-2',3'-O*-isopropylidène-5'-O-acétyl-β-D-ribofuranose.

De façon particulièrement avantageuse, les composés selon l'invention sont choisis parmi :
- 1'-(4-ethoxycarbonyl-5-(2-thienyl)-[1,2,3]-triazol-1-yl)-2',3',5'-tri-O-acétyl- β-D-ribofuranose;
- 1'-(4-ethoxycarbonyl-5-éthylpropiolate-[1,2,3]-triazol-1-yl)-2',3',5'-tri-O-acétyl-β-D-ribofuranose;
- 1'-(4-ethoxycarbonyl-5-éthylpropiolate-[1,2,3]-triazol-1-yl)-2',3'-O-isopropylidène-β-D-ribofuranose;
- 1'-(4-ethoxycarbonyl-5-éthylpropiolate-[1,2,3]-triazol-1-yl)-2',3'-O-isopropylidène-5'-O-acétyl-β-D-ribofuranose; et
- 1-(4-méthylbenzyl)-4-ethoxycarbonyl-5-éthylpropiolate-1,2,3-triazole.

Plus préférentiellement encore, les composés selon l'invention sont choisis parmi :
- 1'-(4-ethoxycarbonyl-5-(2-thienyl)-[1,2,3]-triazol-1-yl)-2',3',5'-tri-O-acétyl-β-D-ribofuranose
- 1'-(4-ethoxycarbonyl-5-phényl-[1,2,3]-triazol-1-yl)-2',3',5'-tri-O-acétyl-β-D-ribofuranose
- 1-(4-méthylbenzyl)-4-ethoxycarbonyl-5-éthylpropiolate-1,2,3-triazole
- 1'-(4-ethoxycarbonyl-5-éthylpropiolate-[1,2,3]-triazol-1-yl)-2',3',4',6'-tétra-O-acétyl-β-D-glucopyranose
- 1'-(4-ethoxycarbonyl-5-(2-thienyl)-[1,2,3]-triazol-1-yl)-2',3'-O-isopropylidène-β-D-ribofuranose.

Les composés divulgués peuvent également se présenter sous forme de pro-drogues. On entend par pro-drogue, une substance pharmacologique (un médicament) qui est administrée sous une forme inactive (ou beaucoup moins active que son métabolite). Une fois administrée, la pro-drogue est métabolisée in vivo en un métabolite actif. Plus particulièrement, les pro-drogues peuvent être classées en deux catégories basées sur leurs sites de conversion dans leur forme active définitive.

Les pro-drogues de type I sont celles où la conversion est intracellulaire. Les pro-drogues de type II sont celles qui sont converties extracellulairement, notamment dans les sucs digestifs ou la circulation systémique.

Les deux types peuvent être encore subdivisés en sous-type A ou B, basés sur des critères supplémentaires. Ceux de type IA et IB se distinguent par le site d'activation qui est le site d'action thérapeutique ou non. Pour le type IIA et IIB, ils sont classés selon que la conversion a lieu dans le tractus gastro-intestinal ou la circulation systémique.

Avantageusement, les composés selon l'invention peuvent comprendre des groupements permettant d'augmenter leur solubilité, leur pénétration cellulaire et leur biodisponibilité.

A titre d'exemples non limitatifs de pro-drogues, on peut citer :
- des pro-drogues contenant des groupements R5, R6 ou R7, ou une combinaison de ceux-ci de type acide aminés ou analogues, sulfates ou phosphoniums de formule générale suivante : 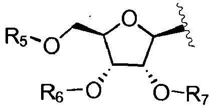 dans laquelle :
- R5 est préférentiellement un acide aminé, du sulfonium ou du phosphonium ;
- R6 et R7 sont préférentiellement des acides aminés identiques ou différents tels que glycine, valine ou leucine.
- des pro-drogues de phosphate ou monophosphates comme les phosphoramidates contenant des groupements R8 et R9, ou une combinaison de ceux-ci-de type acide aminés ou analogues amines, alcools, ou soufrés de formule générale : 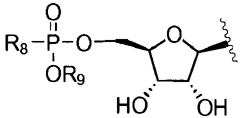 dans laquelle :
- R8 est préférentiellement un acide aminé tel que l'alanine, la leucine ou la valine ou des analogues d'amine ;
- R9 est un phényle ou un analogue de phényle tel que CH₃Ph ou CH₃OPh.

Les différents composés précédemment décrits peuvent être synthétisés selon les différents procédés décrits ci-dessous. Dans ces procédés, les groupements R₁, R₂ et R₃ sont tels que précédemment définis.

Selon une première alternative, les composés de l'invention sont obtenus par une réaction en one-pot, dans laquelle un azide (R₁N₃) réagit avec un alcyne (R₂-C≡C-H) et un électrophile (R₃-X, pour lequel X= Br ou I) en présence de cuivre (catalyseur) pour conduire aux produits triazoles trisubstitués, selon le schéma réactionnel suivant :

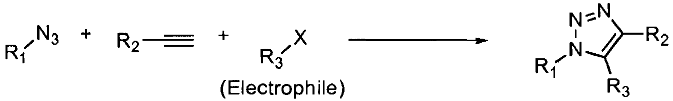

Selon une deuxième alternative, les composés selon l'invention sont obtenus par une réaction de Sonogashira, dans laquelle des substrats triazoles (X= Br ou I) sont mis à réagir en présence d'alcynes (R₄-C≡C-H) et d'un catalyseur au palladium tel que par exemple Pd(PPh₃)₄ ou Pd(PPh₃)₂Cl₂). Cette réaction permet d'obtenir des dérivés 1,2,3-triazoles 1,4,5-trisubstitués (contenant R₁, R₂ et un groupement R₄-C≡C-en position 5 entrant dans la définition de R₃ précisée ci-dessus, pour lequel R₄ = H, SiMe₃, Ph, CO₂Me, CO₂Et), selon le schéma réactionnel suivant :

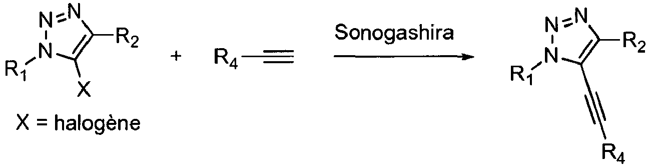

Selon une troisième alternative, les composés selon l'invention sont obtenus par une réaction de couplage au palladium de type-Stille (ou Suzuki-Miyaura). Cette réaction permet de coupler un dérivé stannique (R₃-SnBu₃) ou un dérivé d'acide boronique (R₃-B(OH)₂) avec un 5-halo-triazole-1,4-trisubstitué (X= Br ou I) afin d'obtenir des 1,2,3-triazoles 1,4,5-trisubstitués contenant en position 5 un groupement R₃ (aryle ou hétéro-aryle), selon le schéma réactionnel suivant :

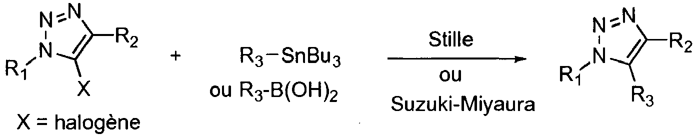

Selon une quatrième alternative, les composés selon l'invention sont obtenus par une réaction entre un azide (R₁N₃) et un alcyne (R₂-C≡C-H) en présence d'un catalyseur à base de cuivre et d'un oxydant (tel que par exemple H₂O₂). Cette réaction permet la synthèse des 1,2,3-triazoles 1,4,5-trisubstitués (contenant R₁, R₂ et le groupement R₂-C≡C- en position 5 entrant dans la définition de R₃ précisée ci-dessus), selon le schéma réactionnel suivant :

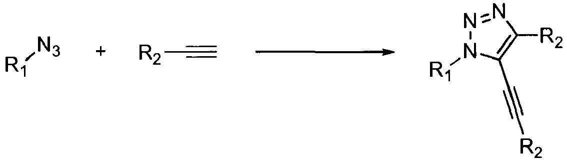

Comme cela est démontré à l'exemple 1, les composés selon l'invention permettent d'agir sur les deux types de morts cellulaires programmées (PCD pour Programmed cell Death) exposées ci-dessus. Parmi les voies de signalisations empruntées par les composés selon l'invention, il apparait que certains composés permettent une inhibition de la voie mTOR.

La protéine mTOR est au centre de nombreux processus métaboliques, tels que notamment la synthèse protéique, la progression en G1, la survie cellulaire ou encore le cytosquelette. De ce fait, la voie de signalisation passant par cette protéine est considérée actuellement comme une nouvelle cible thérapeutique de grand intérêt pour le traitement de nombreux cancers et maladies métaboliques.

A titre d'exemple non limitatif de maladies métaboliques pour lesquelles les composés selon l'invention apparaissent adaptés, on peut citer le diabète, les tumeurs solides notamment les carcinomes de toutes origines incluant par exemple les tumeurs du colon, du sein, de la prostate et les mélanomes.

De façon préférentielle, les composés selon l'invention sont adaptés aux traitements des cancers, c'est-à-dire pour des applications en cancérologie et/ou en onco-hématologie.

A titre d'exemples non limitatifs de traitements pour lesquels les composés sont adaptés, on peut citer le traitement des hémopathies malignes telles que les leucémies chroniques (LMC, LLC), les leucémies aiguës myéloblastiques (LAM), les syndromes Myélodysplasiques (SMD) de haut risque, le Myélome Multiple, le traitement des résistances à l'Imatinib et aux inhibiteurs de tyrosine kinase de seconde génération dans le cas de la LMC et du Vidaza (5-Aza) dans le cas des syndromes myélodysplasiques (SMD). Les composés selon l'invention trouvent également des applications pour le traitement des cancers épithéliaux et des maladies métaboliques. Ils peuvent également être utilisés pour circonvenir les résistances aux autres médicaments, notamment au Glivec™.

De façon particulière, les composés selon l'invention sont adaptés au traitement:
- des patients adultes et enfants atteints de leucémie myéloïde chronique (LMC) chromosome Philadelphie (bcr-abl) positive (Ph+) nouvellement diagnostiquée lorsque la greffe de moelle osseuse ne peut être envisagée comme un traitement de première intention ;
- des patients adultes et enfants atteints de LMC Ph+ en phase chronique après échec du traitement par l'interféron alpha, ou en phase accélérée ou en crise blastique ;
- des patients adultes atteints de leucémie aiguë lymphoïde chromosome Philadelphie positive (LAL Ph+) nouvellement diagnostiquée en association avec la chimiothérapie ;
- des patients adultes atteints de LAL Ph+ réfractaire ou en rechute en monothérapie ;
- des patients adultes atteints de syndromes myélodysplasiques/myéloprolifératifs (SMD/SMP) associés à des réarrangements du gène du PDGFR (platelet-derived growth factor receptor) ;
- des patients adultes atteints d'un syndrome hyperéosinophilique (SHE) à un stade avancé et/ou d'une leucémie chronique à éosinophiles (LCE) associés à un réarrangement du FIP1L1-PDGFRα ;
- des patients adultes atteints de tumeurs stromales gastro-intestinales (GIST - gastrointestinal stromal tumours) malignes Kit (CD 117) positives non résécables et/ou métastatiques ;
- des patients adultes présentant un risque significatif de rechute après résection d'une tumeur stromale gastro-intestinale GIST Kit (CD117) positive. Les patients qui présentent un faible ou très faible risque ne doivent pas être traités ; et
- des patients adultes atteints de dermatofibrosarcome protuberans (DFSP ou maladie de Darier-Ferrand) non résécable et patients adultes atteints de DFSP en rechute et/ou métastatique ne relevant pas d'un traitement chirurgical.

De façon avantageuse, les composés selon l'invention sont particulièrement adaptés au traitement des cancers, notamment les cancers résistants aux inhibiteurs de tyrosine kinases (ITK).

De façon particulièrement avantageuse, les composés selon l'invention sont particulièrement adaptés au traitement de la leucémie myéloïde chronique (LMC), notamment les LMC résistantes aux inhibiteurs de tyrosine kinases (ITK) tels que par exemple l'Imatinib, le Dasatinib et le Nilotinib couramment utilisés dans le traitement de cette pathologie.

Un autre objet de l'invention concerne un produit pharmaceutique contenant un composé selon l'invention et au moins un deuxième actif comme produit de combinaison pour une administration simultanée, séparée ou étalée dans le temps, pour son utilisation dans le traitement du cancer.

Avantageusement, le deuxième principe actif est un agent anti-tumoral, un agent anti-inflammatoire, ou un agent diminuant les effets secondaires liés aux composés selon l'invention.

De préférence, le deuxième actif est un composé anti-tumoral choisi parmi les agents alkylants, les anti-métabolites, les alcaloïdes végétaux, les inhibiteurs de la topoisomérase, et les antibiotiques antitumoraux.

A titre d'exemple non limitatif d'agent anti-tumoral utilisable selon l'invention, on peut citer notamment le bortezomib, la cisplatine, la carboplatine, l'ifosfamide, le chlorambucil, le busulfan, le thiotépa, le 5-fluoro-uracile (5FU), la fludarabine, le méthotrexate, la vincristine, la vinblastine, la vinorelbine, le paclitaxel, le docétaxel, les dérivés de la camptothécine, l'amsacrine, les anthracyclines, les dérivés de l'épipodophyllotoxine, la doxorubicine, la daunorubicine, l'actinomycine D, la mitomycine C, la plicamycine et la bléomycine. On peut également citer les ITK utilisés dans différentes pathologies tumorales comme par exemple Imatinib, Dasatinib, Nilotinib et Sunitinib. De façon alternative, le produit de combinaison selon l'invention comprend une association d'un composé selon l'invention et d'au moins deux autres agents anti-tumoraux, ou au moins trois autres agents anti-tumoraux ou plus.

Par utilisation thérapeutique simultanée, au sens de la présente invention, on entend une administration d'au moins deux principes actifs par la même voie et au même moment ou sensiblement au même moment.

Par utilisation thérapeutique séparée, au sens de la présente invention, on entend notamment une administration d'au moins deux principes actifs au même moment ou sensiblement au même moment par des voies différentes.

Par utilisation thérapeutique étalée dans le temps, on entend une administration d'au moins deux principes actifs à des moments différents, la voie d'administration étant identique ou différente.

Plus particulièrement, on entend un mode d'administration selon lequel l'ensemble de l'administration de l'un des principes actifs est effectué avant que l'administration de l'autre ou des autres ne commence.

On peut ainsi administrer l'un des principes actifs pendant plusieurs mois avant d'administrer l'autre ou les autres principes actifs. Il n'y a pas de traitement simultané dans ce cas. On peut aussi envisager une administration alternée de chaque principe actif pendant plusieurs semaines.

Un autre objet de l'invention concerne une composition pharmaceutique comprenant un composé selon l'invention et un support pharmaceutiquement acceptable.

La voie d'administration de la composition selon l'invention peut être par voie orale, parentérale, topique, oculaire. De préférence, la composition pharmaceutique est conditionnée sous une forme convenant à une application par voie orale.

Ainsi, par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

De façon alternative, lorsque la composition selon l'invention est administrée par voie parentérale, elle peut se présenter sous forme de solutions ou suspensions pour perfusion ou pour injection intramusculaire, intraveineuse ou sous-cutanée.

De façon alternative encore, lorsque la composition selon l'invention est administrée par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de solutions aqueuses, hydroalcooliques ou huileuses, de dispersions du type lotion, de gels aqueux, anhydres ou lipophiles, de poudres, de tampons imbibés, de syndets, de lingettes, de sprays, de mousses, de sticks, de shampoings, de compresses, de bases lavantes, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-liquide ou solide du type crème, gel ou pommade. Elle peut également se présenter sous forme de suspensions de microsphères ou nanosphères ou de vésicules lipidiques ou polymériques ou de patchs polymériques ou gélifiés permettant une libération contrôlée.

Suite à la réalisation d'études de stabilité et de solubilité, il apparait que les composés selon l'invention sont particulièrement stables et solubles dans des solutions comprenant :
- du DMA (Diméthylacétamide) ;
- du Tween 60 Polysorbate 60 (Polyoxyéthylène (20) sorbitan Monostéarate) ou du Tween 80 (Polyoxyéthylène (20) sorbitan monooléate) ; et
- du PBS (tampon phosphate salin) ou de l'eau (H₂O).

De préférence, les composés selon l'invention sont solubilisés dans une composition comprenant :
- 2% de DMA (Diméthylacétamide) en poids du poids total de la composition ;
- 5% de Tween 80 (Polyoxyéthylène (20) sorbitan monooléate) en poids du poids total de la composition ; et
- 93% de PBS (tampon phosphate salin) en poids du poids total de la composition.

Selon l'invention, le sujet à traiter est préférentiellement un mammifère. Préférentiellement encore, il s'agit d'un humain, d'un cheval, d'un chien ou d'un chat. Plus préférentiellement encore, le patient à traiter est un humain.

### - Exemples -

### Exemple 1 :

Seize nouveaux composés, analogues de l'AICAR (acadésine), ont été évaluées sur deux types de lignées leucémie myéloïde chronique (LMC) : sensibles (K562) ou résistantes (ImaR) à l'Imatinib. Il s'agit des 16 composés suivants repris dans le tableau 1 ci-dessous.

**Tableau 1 :**

| Code molécule | Formule chimique de la molécule | Nom chimique de la molécule |
|---|---|---|
| 15 | 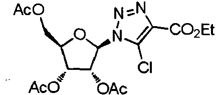 | 1'-(4-ethoxycarbonyl-5-chloro-[1,2,3]-triazol-1-yl)-2',3',5'-tri-O-acétyl-β-D-ribofuranose |
| 17 | 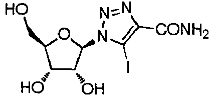 | 1'-(4-carbamoyl-5-iodo-[1,2,3]-triazol-1-yl)-β-D-ribofuranose |
| 23 | 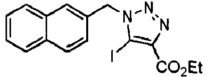 | 1-(naphtyl-2-methyl)-4-ethoxycarbonyl-5-iodo-1,2,3-triazole |
| 82 | 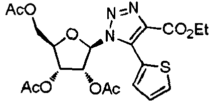 | 1'-(4-ethoxycarbonyl-5-(2-thienyl)-[1,2,3]-triazol-1-yl)-2',3',5'-tri-O-acétyl-β-D-ribofuranose |
| 86 | 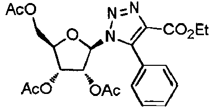 | 1'-(4-ethoxycarbonyl-5-phényl-[1,2,3]-triazol-1-yl)-2',3',5'-tri-O-acétyl-β-D-ribofuranose |
| 191 | 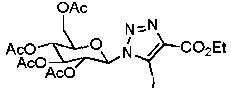 | 1'-(4-ethoxycarbonyl-5-iodo-[1,2,3]-triazol-1-yl)-2',3',4',6'-tétra-O-acétyl-β-D-glucopyranose |
| 49 | 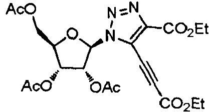 | 1'-(4-ethoxycarbonyl-5-éthylpropiolate-[1,2,3]-triazol-1-yl)-2',3',5'-tri-O-acétyl-β-D-ribofuranose |
| 115 | 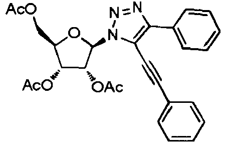 | 1'-(4-phényl-5-phénylacétylene-[1,2,3]-triazol-1-yl)-2',3',5'-tri-O-acétyl-β-D-ribofuranose |
| 172 | 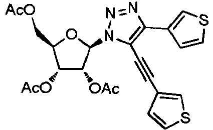 | 1'-(4-(3-thienyl)-5-(3-ethynylthiophene)-[1,2,3]-triazol-1-yl)-2',3',5'-tri-O-acétyl-β-D-ribofuranose |
| 176 | 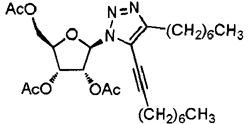 | 1'-(4-heptyl-5-(non-1-yn-1-yl)-[1,2,3]-triazol-1-yl)-2',3',5'-tri-O-acétyl-β-D-ribofuranose |
| 177 | 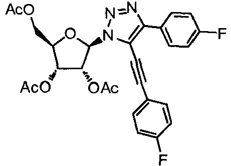 | 1'-(4-(4-fluorophényl)-5-(4-fluorophénylacétylene)-[1,2,3]-triazol-1-yl)-2',3',5'-tri-O-acétyl-β-D-ribofuranose |
| 25 | 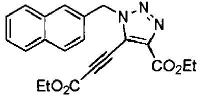 | 1-(naphtyl-2-methyl)-4-ethoxycarbonyl-5-éthylpropiolate-1,2,3-triazole |
| 97 | 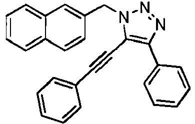 | 1-(naphtyl-2-methyl)-4-phényl-5-phénylacétylene-1,2,3-triazole |
| 112 | 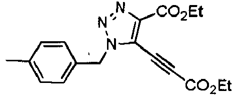 | 1-(4-méthylbenzyl)-4-ethoxycarbonyl-5-éthylpropiolate-1,2,3-triazole |
| 114 | 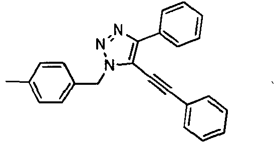 | 1-(4-méthylbenzyl)-4-phényl-5-phénylacétylene-1,2,3-triazole |
| 184 | 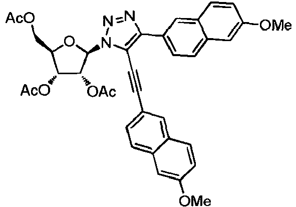 | 1'-(4-(6-méthoxynapht-2-yl)-5-((6-méthoxynapht-2-yl)acétylene)-[1,2,3]-triazol-1-yl)-2',3',5'-tri-O-acétyl-β-D-ribofuranose |

La lignée de cellules leucémiques K562 est obtenue à partir de l'épanchement pleural d'une patiente atteinte de LMC. Cette lignée constitue un bon modèle pour l'étude de cette maladie. Les cellules ImaR résistantes à l'Imatinib sont générées à partir des cellules K562 par exposition à des doses croissantes d'Imatinib. Les cellules K562 et ImaR sont cultivées a 37°C sous 5% de CO2 dans un milieu RPMI 1640 contenant 5% de sérum de veau foetal et supplémenté en pyruvate de sodium et en antibiotiques (Pénicilline Streptomycine).

### a. Criblage des 16 composés par mesure de la viabilité cellulaire :

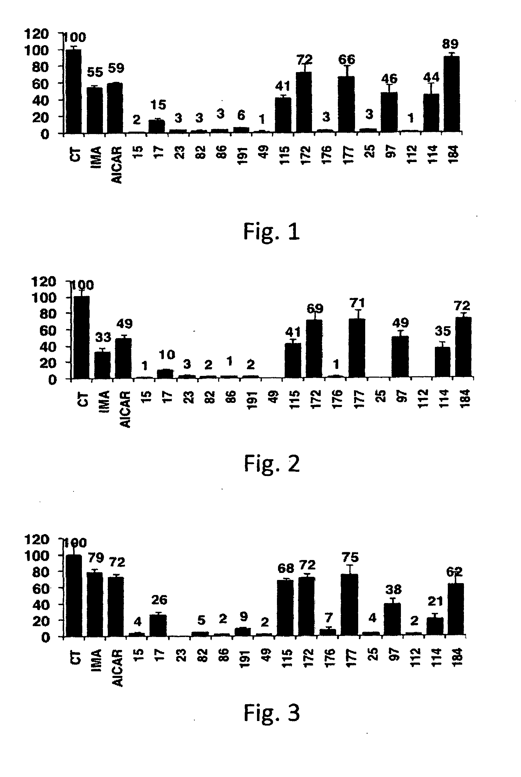

Afin de cribler les 16 composés selon l'invention, l'activité métabolique des cellules K562 et ImaR est étudiée après 24 ou 48 heures de traitement par 0,5 mM (dose efficace d'AICAR) de ces différents composés. Les figures 1 à 4 reflètent l'étude de la viabilité cellulaire.

La figure 1 représente le pourcentage de cellules K562 sensibles à l'Imatinib (K562) viables après stimulation ou non pendant 24h avec de l'Imatinib (1 µM), de l'AICAR ou l'un des 16 composés différents selon l'invention (0,5 mM) décrits dans le tableau 1 ci-dessus en plaque 96 puits. Après ajout de réactif XTT, l'absorbance est mesurée à 490nm.

La figure 2 représente le pourcentage de cellules K562 sensibles à l'Imatinib (K562) viables après stimulation ou non pendant 48h avec de l'Imatinib (1 µM), de l'AICAR ou l'un des 16 composés différents selon l'invention (0,5 mM) décrits dans le tableau 1 ci-dessus en plaque 96 puits. Après ajout de réactif XTT, l'absorbance est mesurée à 490nm.

La figure 3 représente le pourcentage de cellules résistantes à l'Imatinib (ImaR) viables après stimulation ou non pendant 24h avec de l'Imatinib (1 µM), de l'AICAR ou l'un des 16 composés différents selon l'invention (0,5 mM) décrits dans le tableau 1 ci-dessus en plaque 96 puits. Après ajout de réactif XTT, l'absorbance est mesurée à 490nm.

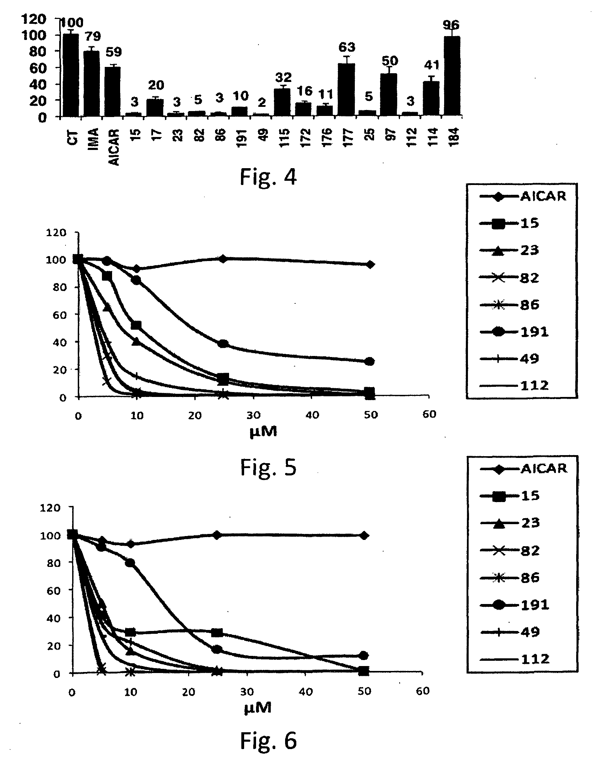

La figure 4 représente le pourcentage de cellules résistantes à l'Imatinib (ImaR) viables après stimulation ou non pendant 48h avec de l'Imatinib (1 µM), de l'AICAR ou l'un des 16 composés différents selon l'invention (0,5 mM) décrits dans le tableau 1 ci-dessus en plaque 96 puits. Après ajout de réactif XTT, l'absorbance est mesurée à 490nm.

Les résultats obtenus montrent que l'ensemble des 16 composés induisent, après 24 ou 48 heures de stimulation, une diminution de la viabilité cellulaire dans les deux types de clones. Cette diminution de la viabilité cellulaire est plus importante que le témoin positif. Elle est également plus importante ou comparable aux composés Imatinib ou AICAR. Ainsi, ces 16 composés apparaissent être des candidats intéressants comme principes actifs destinés au traitement de la LMC.

En outre, parmi ces 16 composés, 9 composés (15, 23, 82, 86, 191, 49, 176, 25, 112) induisent, après 24 ou 48 heures de stimulation et de manière quasiment identique dans les deux types de clones, une importante diminution de la viabilité cellulaire. En effet, le pourcentage de cellules viables passe dès 24 heures de traitement à des valeurs inférieures à 10%. Par ailleurs et comme attendu, l'Imatinib induit chez la lignée K562 une perte de viabilité cellulaire de plus de 50% après 48 heures de stimulation tandis que pour la lignée ImaR, la viabilité cellulaire n'est que faiblement affectée.

L'AICAR, en revanche, entraîne une perte de viabilité cellulaire quel que soit le clone considéré (Fig. 1A, B). 7 composés préférés sont ensuite utilisées dans des expériences de dose-réponse (concentrations croissantes de 5 à 50 µM pour 24 ou 48 heures de traitement) sur les deux types de clones (Figures 1 à 4).

### b. Expérience de dose-réponse de 7 composés :

La figure 5 représente une expérience dose-réponse sur des cellules K562 exposées pendant 24h à de l'Imatinib (1 µM) (pour une dose-réponse complète des effets de l'Imatinib sur les cellules K562S et K562R voir Jacquele et al, Oncogene 2007, 26, 2445-2458) ou à des doses croissantes (5, 10, 25 et 50 µM) d'AICAR ou d'un des 7 composés choisis parmi les composés 15, 23, 82, 86, 191, 49 et 112.

La figure 6 représente une expérience dose-réponse sur des cellules K562 exposées pendant 48h à de l'Imatinib (1 µM) (pour une dose-réponse complète des effets de l'Imatinib sur les cellules K562S et K562R voir Jacquele et al, Oncogene 2007, 26, 2445-2458) ou à des doses croissantes (5, 10, 25 et 50 µM) d'AICAR ou d'un des 7 composés choisis parmi les composés 15, 23, 82, 86, 191, 49 et 112.

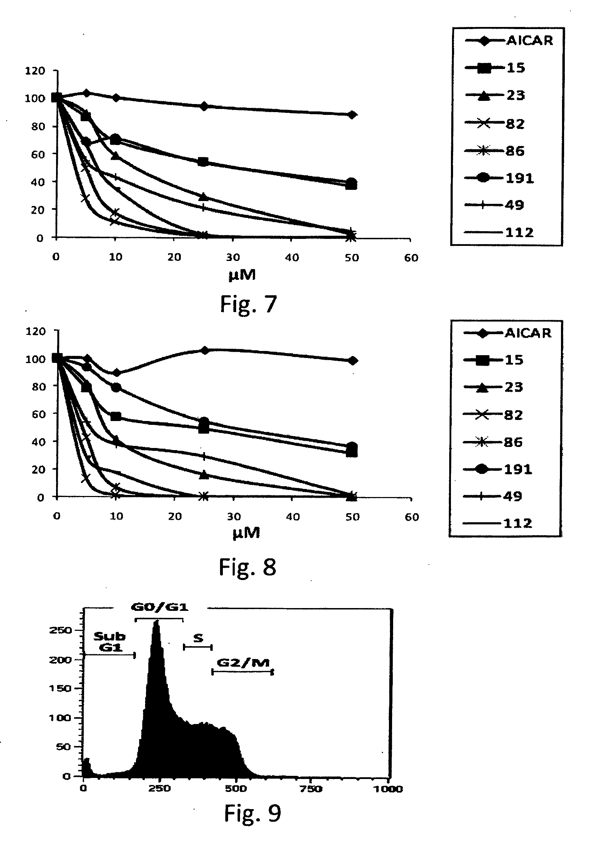

La figure 7 représente une expérience dose-réponse sur des cellules ImaR exposées pendant 24h à de l'Imatinib (1 µM) (pour une dose-réponse complète des effets de l'Imatinib sur les cellules K562S et K562R voir Jacquele et al, Oncogene 2007, 26, 2445-2458) ou à des doses croissantes (5, 10, 25 et 50 µM) d'AICAR ou d'un des 7 composés choisis parmi les composés 15, 23, 82, 86, 191, 49 et 112.

La figure 8 représente une expérience dose-réponse sur des cellules ImaR exposées pendant 48h à de l'Imatinib (1 µM) (pour une dose-réponse complète des effets de l'Imatinib sur les cellules K562S et K562R voir Jacquele et al, Oncogene 2007, 26, 2445-2458) ou à des doses croissantes (5, 10, 25 et 50 µM) d'AICAR ou d'un des 7 composés choisis parmi les composés 15, 23, 82, 86, 191, 49 et 112.

Les courbes des figures 5 à 8 illustrent l'effet dose-réponse de chacun de ces 7 composés. L'AICAR, inefficace à ces concentrations, sert de référence. Après ajout de réactif XTT, l'absorbance est mesurée à 490 nm.

Les résultats illustrés aux figures 5 à 8 montrent une diminution dose-dépendante du nombre de cellules viables pour les 7 composés dès 24 heures de stimulation. Ces 7 composés se révèlent particulièrement efficaces sur les deux lignées à des concentrations aussi faibles que 5 µM (15, 23, 82, 86, 191, 49, 112). L'IC50 est d'environ 2,5 µM pour les composés 82, 86, 112 et d'environ 10 µM pour les composés 15, 23, 191, 49. En comparaison, l'AICAR, à ces mêmes concentrations, est inefficace. Enfin, le clone K562 semble légèrement plus sensible à ces composés que le clone ImaR.

### c. Analyse par cytométrie de flux du cycle cellulaire :

Le pourcentage de cellules dans chacune des phases du cycle cellulaire (subG1, cellules en apoptose, G0/G1, S et G2/M, Fig. 3A) est déterminé par cytométrie de flux, suite à l'incubation des deux lignées de cellules avec de l'Imatinib, de l'AICAR ou les composés 82, 86, 112, 15, 23, 191, 49.

La figure 9 illustre un modèle de distribution des cellules dans chacune des phases du cycle cellulaire (subG1, G0/G1, S, G2/M). L'abscisse du graphe de la figure 9 correspond à la quantité d'ADN marqué à l'iodure de propidium tandis l'ordonnée correspond au nombre de cellules.

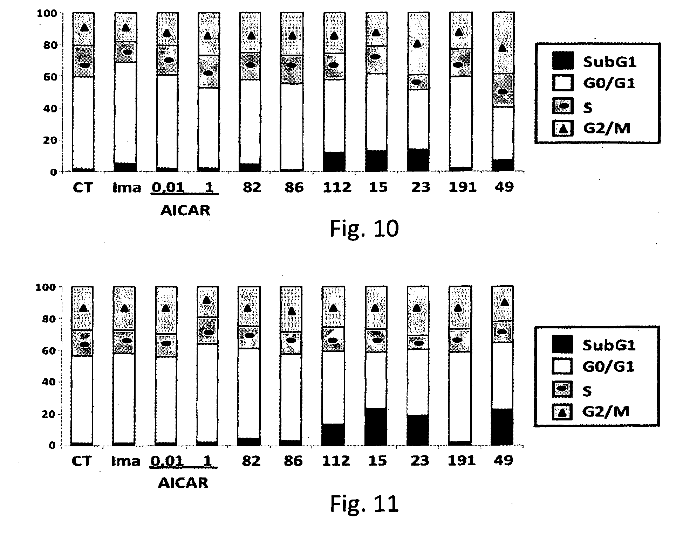

La figure 10 représente le pourcentage de cellules dans chacune des phases du cycle cellulaire (ordonnée) lorsque des cellules K562 sont stimulées pendant 24h avec de l'Imatinib (3 µM), de l'AICAR (0,01 mM et 1 mM), avec les composés 82 (2,5 µM), 86 (2,5 µM), 112 (2,5 µM) ou avec les composés 15 (10 µM), 23 (10 µM), 191 (10 µM) et 49 (10 µM). Les noyaux sont marqués par l'iodure de propidium puis la proportion de cellules qui se trouvent dans les différentes phases du cycle cellulaire (subG1, G0/G1, S, G2/M) est déterminée.

La figure 11 représente le pourcentage de cellules dans chacune des phases du cycle cellulaire (ordonnée) lorsque des cellules ImaR sont stimulées pendant 24h avec de l'Imatinib (3 µM), de l'AICAR (0,01 mM et 1 mM), avec les composés 82 (2,5 µM), 86 (2,5 µM), 112 (2,5 µM) ou avec les composés 15 (10 µM), 23 (10 µM), 191 (10 µM) et 49 (10 µM). Les noyaux sont marqués par l'iodure de propidium puis la proportion de cellules qui se trouvent dans les différentes phases du cycle cellulaire (subG1, G0/G1, S, G2/M) est déterminée.

Les résultats illustrés aux figures 10 et 11 montrent une augmentation du pourcentage de cellules dans la phase G0/G1 du cycle cellulaire après 24 heures de traitement par l'Imatinib. Une petite fraction de cellules s'accumule aussi au stade subG1 ce qui suggère un phénomène d'apoptose. Comme attendu, ces effets ne s'observent pas chez le clone ImaR. En ce qui concerne l'AICAR, il semble provoquer l'arrêt du cycle cellulaire en G2/M pour le clone K562 et en G0/G1 pour les cellules ImaR. Enfin, 5 des 7 composés (82, 86, 15, 23, 49) induisent une augmentation de la proportion de cellules K562 et ImaR dans la phase G2/M, accompagnée pour les composés 112, 15, 23 et 49 d'une accumulation très prononcée de cellules dans la phase subG1 et d'une diminution du nombre de cellules en G0/G1.

Les composés induisent un arrêt des cellules K562-S et K562-R en phase G2/M du cycle cellulaire qui s'accompagne ou non d'une apoptose cellulaire.

### d. Analyse de l'activation des caspases 3, 8 et 9 dans les cellules K562 et ImaR :

### i) Mesure de l'activité de la caspase 3 :

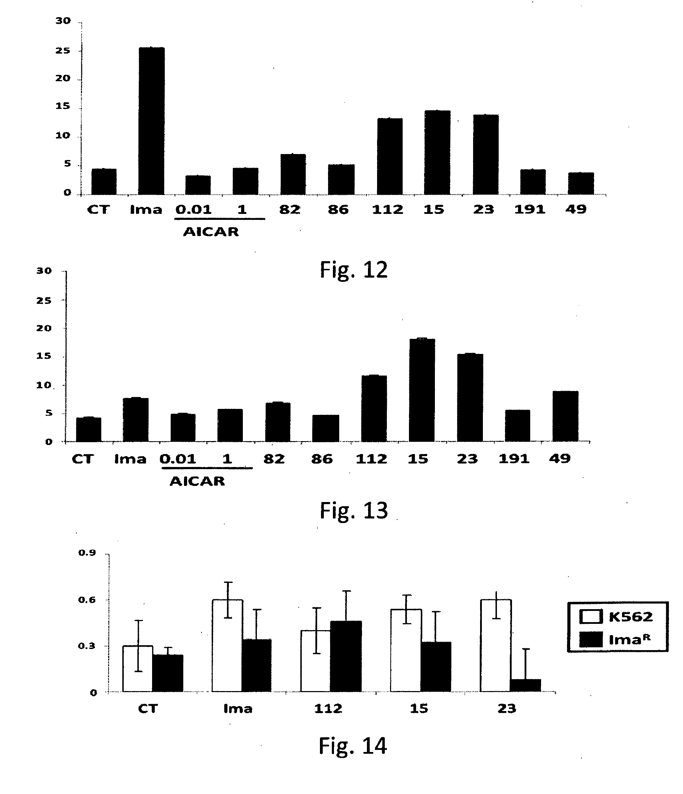

La figure 12 illustre l'activité de la caspase 3 en U.A/mg de protéine (ordonnée) lorsque des cellules K562 sensibles à l'Imatinib sont stimulées pendant 24h avec de l'Imatinib (3 µM), de l'AICAR (0,01 mM et 1 mM), les composés 82 (2,5 µM), 86 (2,5 µM), 112 (2,5 µM) ou avec les composés 15 (10 µM), 23 (10 µM), 191 (10 µM) et 49 (10 µM). Les cellules sont lysées et 10 µg de protéines sont mis en présence d'Ac-DEVD-AMC en plaque 96 puits. L'émission de fluorescence à 460 nm résultant de la libération de l'AMC est ensuite enregistrée.
La figure 13 illustre l'activité de la caspase 3 en U.A/mg de protéine (ordonnée) lorsque des cellules ImaR résistantes à l'Imatinib sont stimulées pendant 24h avec de l'Imatinib (3 µM), de l'AICAR (0,01 mM et 1 mM), les composés 82 (2,5 µM), 86 (2,5 µM), 112 (2,5 µM) ou avec les composés 15 (10 µM), 23 (10 µM), 191 (10 µM) et 49 (10 µM). Les cellules sont lysées et 10 µg de protéines sont mis en présence d'Ac-DEVD-AMC en plaque 96 puits. L'émission de fluorescence à 460 nm résultant de la libération de l'AMC est ensuite enregistrée.

Les résultats de ces figures 12 et 13 montrent que le traitement des cellules K562 par 3 µM d'Imatinib pendant 24 heures n'induit quasiment pas d'augmentation de l'activité de la caspase 3 dans les cellules ImaR alors qu'elle est augmentée d'un facteur 4 environ dans les cellules K562. L'AICAR (1 mM), en revanche, est incapable d'induire cette activation quel que soit le clone considéré. Ces résultats confirment les effets observés lors de l'analyse du cycle cellulaire par cytométrie de flux : les composés 112, 15, 23 induisent une augmentation de l'activité de la caspase 3 d'un facteur allant de 2 à 3 par rapport au contrôle dans les cellules K562 et ImaR. De façon intéressante, le composé 49 induit une augmentation de l'activité de la caspase 3 dans le clone ImaR mais pas dans les cellules K562.

Ces résultats conduisent à mesurer l'activation des caspases 8 et 9 pour ces trois composés 112, 15, 23.

### ii) Mesure des activités des caspases 8 et 9 :

La figure 14 illustre l'activité de la caspase 8 en U.A/mg de protéine (ordonnée) lorsque des cellules K562 sensibles à l'Imatinib (barres blanches) ou des cellules ImaR résistantes à l'Imatinib (barres noires) sont stimulées pendant 24h avec de l'Imatinib (3 µM), avec les composés 112 (2,5 µM), 15 (10 µM) ou 23 (10 µM). Les cellules sont lysées et les activités de caspase 8 sont mesurées en utilisant 10 µg de protéines et Ac-IETD-AMC. L'émission de fluorescence à 460 nm résultant de la libération de l'AMC est ensuite enregistrée.

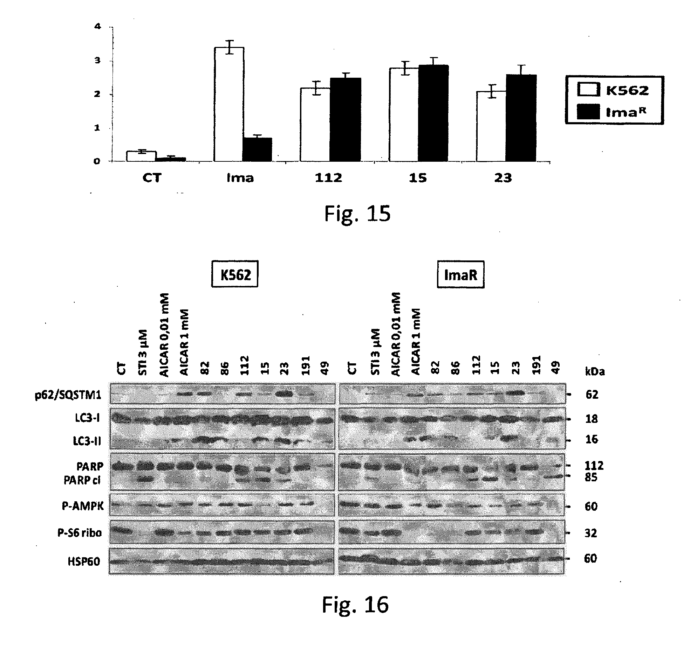

La figure 15 illustre l'activité de la caspase 9 en U.A/mg de protéine (ordonnée) lorsque des cellules K562 sensibles à l'Imatinib (barres blanches) ou des cellules ImaR résistantes à l'Imatinib (barres noires) sont stimulées pendant 24h avec de l'Imatinib (3 µM), avec les composés 112 (2,5 µM), 15 (10 µM) ou 23 (10 µM). Les cellules sont lysées et les activités de caspase 9 sont mesurées en utilisant 10 µg de protéines et Ac-LEHD-AMC. L'émission de fluorescence à 460 nm résultant de la libération de l'AMC est ensuite enregistrée.

Les résultats illustrés aux figures 14 et 15 illustrent qu'aucune induction significative de l'activité de la caspase 8 n'est mesurée en présence des différents composés (Fig. 14). En revanche, le traitement des cellules K562 par l'Imatinib induit une forte augmentation de l'activité de la caspase 9 ce qui n'est pas le cas dans les cellules ImaR. Les composés 112, 15, 23 induisent eux aussi, et de façon quasi similaire dans les deux types de clones, l'activation de cette caspase (Fig. 15).

### e. Analyse de l'expression et de la phosphorylation de marqueurs spécifiques de l'apoptose et de l'autophagie et de l'état d'activation de la voie mTOR :

### i) Analyse de l'expression et de la phosphorylation de marqueurs spécifiques de l'apoptose et de l'autophagie :

La figure 16 représente une analyse par Western Blot de l'expression et de la phosphorylation de marqueurs spécifiques de l'apoptose et de l'autophagie. Les cellules K562 et ImaR sont stimulées pendant 24h avec de l'Imatinib (3 µM), de l'AICAR (0,01 mM et 1 mM), les composés 82 (2,5 µM), 86 (2,5 µM), 112 (2,5 µM) ou les composés 15 (10 µM), 23 (10 µM), 191 (10 µM) et 49 (10 µM). Les cellules sont lysées et 35µg de protéines sont séparées par SDS-PAGE (gel de polyacrylamide à 12 ou 14%) et analysées par Western Blot avec des anticorps spécifiques de chacun des marqueurs.

Les résultats du Western Blot repris à la figure 16 permettent de discriminer le type de mort cellulaire programmée (PCD pour Programmed Cell Death) impliqué dans la cytotoxicité des composés 82, 86, 112, 15, 23, 191, 49, l'expression et la phosphorylation de marqueurs spécifiques de l'apoptose et de l'autophagie. Les résultats obtenus révèlent une augmentation de l'expression de p62/SQSTM1, intervenant dans les étapes précoces de l'autophagie, dans les cellules K562 et ImaR traitées par l'AICAR (1 mM) et les composés 82, 86, 112, 15, 23, 191. Au cours de l'autophagie, LC3-I est clivée en LC3-II puis cette forme se lie à la phosphatidyléthanolamine pour s'insérer dans la membrane du phagophore et permettre l'élongation de l'autophagosome. Cette agrégation de la LC3-I en LC3-II, visualisée en Western blot, représente donc un excellent moyen de démontrer une induction de l'autophagie. LC3 est clivée dans les deux types de clones en présence d'AICAR (1 mM) ou des composés 82, 86, 15, 23, 191, 49. Par ailleurs, durant l'apoptose, un certain nombre de protéines sont clivées par les caspases. Parmi celles-ci, PARP constitue le principal substrat de la caspase 3. Dans les deux types de clones exposés à de l'Imatinib ou aux composés 112, 15, 23, 49, on remarque l'apparition du fragment de clivage caractéristique à 85 kDa (PARP cl). Ces observations confirment donc les résultats précédents.

Enfin, pour déterminer l'implication de la voie AMPK/mTOR dans l'initiation du processus autophagique, le niveau de phosphorylation de la P-AMPK, la protéine AMPK (visualisée par un retard de migration sur le gel) et de P-S6 ribo, la protéine S6 ribosomale, substrat indirect de mTOR ont été visualisés. De manière intéressante, il est constaté que l'AMPK est activée en présence d'1 mM d'AICAR et des différents composés d'intérêt. Cette activation est corrélée avec une inhibition de la voie mTOR visualisée par une déphosphorylation massive de la protéine S6 ribosomale.

Pour confirmer que la voie mTOR est inhibée en présence des différents composés, la phosphorylation d'un substrat direct de mTOR a été réalisé : la protéine 4E-BP1.

### ii) Analyse de l'état d'activation de la voie mTOR

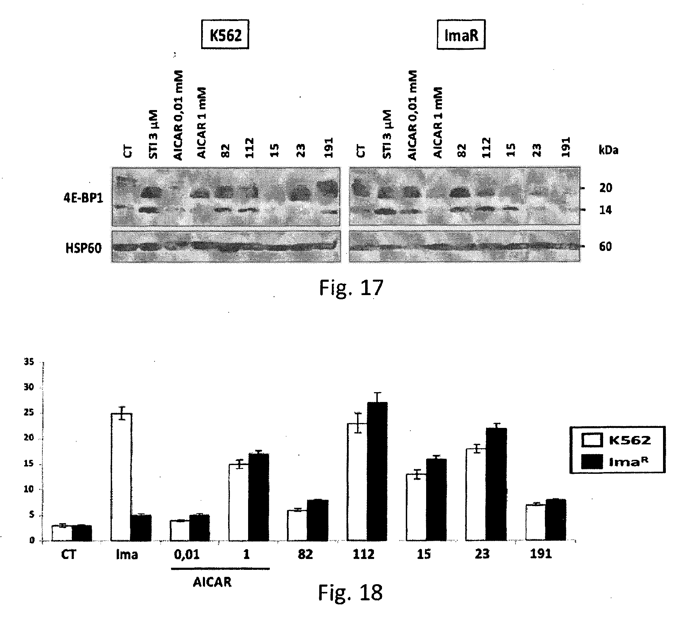

La figure 17 représente une analyse par Western Blot de l'état d'activation de la voie mTOR par étude de la phosphorylation de 4E-BP1, qui est un substrat direct de mTOR. Les cellules K562 et ImaR sont stimulées pendant 24h avec de l'Imatinib (3 µM), de l'AICAR (0,01 mM et 1 mM), les composés 82 (2,5 µM), 112 (2,5 µM) ou les composés 15 (10 µM), 23 (10 µM) et 191 (10 µM). Les cellules sont lysées et 30µg de protéines sont séparées par SDS-PAGE (gel de polyacrylamide à 13%) et analysées par Western Blot avec un anticorps spécifique de la protéine 4E-BP1. Le retard de migration sur gel observé reflète le niveau de phosphorylation de 4E-BP1.

Le Western blot révèle un retard de migration sur gel plus ou moins prononcé suivant son niveau de phosphorylation. Ainsi, une diminution totale de la phosphorylation de 4E-BP1 dans les deux clones sensibles et résistants traités par l'AICAR (1 mM) et par les composés 82 et 23 ainsi qu'une diminution partielle avec le reste des composés est observée. Comme attendu, l'Imatinib inhibe totalement la phosphorylation de 4E- BP1 dans la lignée K562 parentale mais pas dans la contrepartie résistante.

### f. Mise en évidence de l'activité de la Cathepsine-B et de l'activation de la Cathepsine-L :

### i) Mesure de la cathepsine-B :

La figure 18 illustre l'activité de la cathepsine B en U.A/mg de protéine (ordonnée) lorsque des cellules K562 sensibles à l'Imatinib (barres blanches) ou des cellules ImaR résistantes à l'Imatinib (barres noires) sont stimulées pendant 24h avec de l'Imatinib (3 µM), de l'AICAR (0,01 mM ou 1 mM) ou avec les composés 82 (2,5 µM), 112 (2,5 µM) ou avec les composés 15 (10 µM), 23 (10 µM) ou 191 (10 µM). Les cellules sont lysées et 5 µg de protéines sont mis en présence de z-RR-AMC en plaque 96 puits. L'émission de fluorescence à 460 nm résultant de la libération de l'AMC est ensuite enregistrée.

Les résultats concernant l'accumulation des protéines p62 et LC3-II ont conduits à étudier l'activité de la Cathepsine-B puis l'activation de la Cathepsine-L, enzymes lysosomales effectrices de l'autophagie intervenant dans la digestion du contenu des autophagolysosomes. Aussi, comme illustré à la figure 18, une augmentation de l'activité de la Cathepsine-B d'un facteur 2 environ (composés 82, 191) ou supérieur à 3 (composés 112, 15, 23) lors des traitements par l'AICAR (1 mM) ou les composés 82, 112, 15, 23, 191 est observée, et ce de manière semblable dans les deux types de clones à chaque fois.

### ii) Mise en évidence de l'activation de la cathepsine-L :

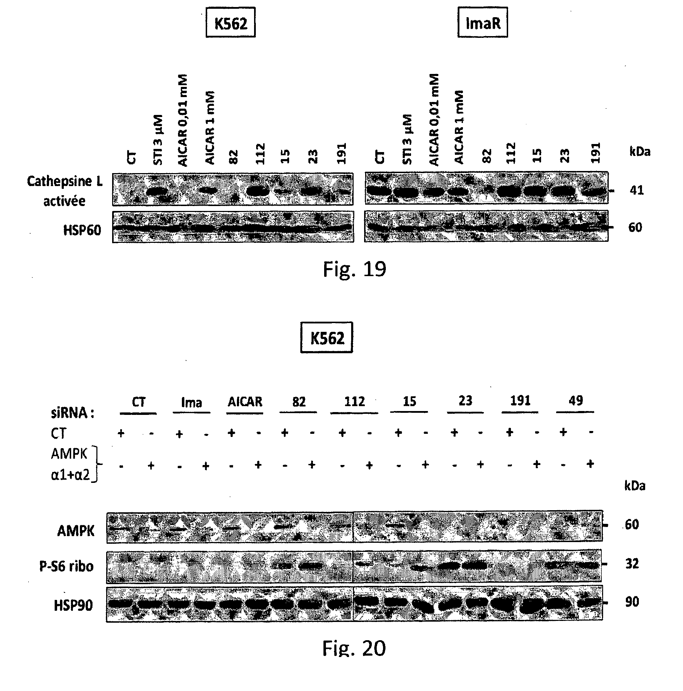

La figure 19 représente une analyse par Western Blot de l'activation de la cathepsine L. Les cellules K562 et ImaR sont stimulées pendant 24h avec de l'Imatinib (3 µM), de l'AICAR (0,01 mM et 1 mM), les composés 82 (2,5 µM), 112 (2,5 µM) ou les composés 15 (10 µM), 23 (10 µM) et 191 (10 µM). Les cellules sont lysées et 30 µg de protéines sont séparées par SDS-PAGE (gel de polyacrylamide à 13%) et analysées par Western Blot avec un anticorps spécifique de la cathepsine L.

L'immunoblot concernant l'activation de la Cathepsine-L illustré à la figure 19 confirme les observations faites lors des mesures de la cathepsine B, puisque l'on remarque l'accumulation de la forme activée de la Cathepsine-L dans les clones K562. De manière intéressante, la Cathepsine-L n'est pas activée dans le clone ImaR et ne semble donc pas participer pas à la lyse du contenu des autophagosomes dans ce clone.

### Conclusion :

D'après les résultats illustrés aux exemples 1 a. à 1 g. ci-dessus, les composés selon l'invention apparaissent présenter une activité anticancéreuse à la fois sur des lignées de cellules leucémiques K562 sensibles ou résistantes à l'Imatinib. Parmi ces 16 composés, les 7 composés les plus efficaces à l'issue des expériences de viabilité cellulaire présentent des caractéristiques particulièrement intéressantes et se révèlent 50 à 500 fois plus puissantes que l'AICAR. Aussi, certains composés semblent induire soit de l'autophagie uniquement (82, 86, 191), soit l'apoptose uniquement (112), soit une combinaison des deux types de mécanismes (15, 23, 49). Cependant, tous ces 7 composés ont pour mécanisme d'action commun une inhibition de la voie mTOR visualisée par une déphosphorylation de tous ses substrats protéiques (figures 16 et 17) et un blocage du cycle cellulaire en phase G2/M (figures 9, 10 et 11). Il est également intéressant de constater que l'apoptose induite par les composés 112, 15, 23 et 49 est essentiellement relayée par la voie intrinsèque nécessitant une perméabilisation mitochondriale et l'activation de la caspase 9.

### Exemple 2 :

13 nouveaux composés, analogues de l'AICAR (acadésine), ont été évalués sur des lignées LMC (leucémie myéloïde chronique) sensibles (K562) à l'Imatinib. :. Il s'agit des 13 composés suivants repris dans le tableau 2 ci-dessous.

**Tableau 2 :**

| Code molécule | Formule chimique de la molécule | Nom chimique de la molécule |
|---|---|---|
| 20 | 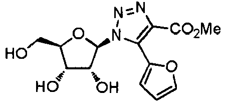 | 1'-(4-méthoxycarbonyl-5-(2-furyl)-[1,2,3]-triazol-1-yl)-p-D-ribofuranose |
| 21 | 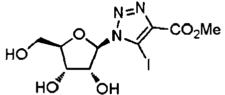 | 1'-(4-méthoxycarbonyl-5-iodo-[1,2,3]-triazol-1-yl)-β-D-ribofuranose |
| 22 | 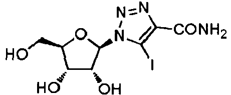 | 1'-(4-carbamoyl-5-iodo-[1,2,3]-triazol-1-yl)-β-D-ribofuranose |
| 23 | 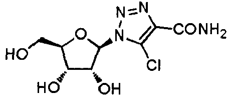 | 1'-(4-carbamoyl-5-chloro-[1,2,3]-triazol-1-yl)-β-D-ribofuranose |
| 24 | 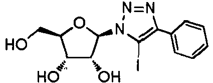 | 1'-(4-phényl-5-iodo-[1,2,3]-triazol-1-yl)-β-D-ribofuranose |
| 25 | 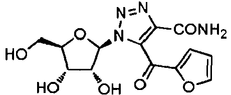 | 1'-(4-ethoxycarbonyl-5-(2-furoyl)-[1,2,3]-triazol-1-yl)-β-D-ribofuranose |
| 26 | 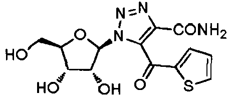 | 1'-(4-ethoxycarbonyl-5-(2-thienoyl)-[1,2,3]-triazol-1-yl)-β-D-ribofuranose |
| 27 | 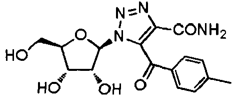 | 1 '-(4-eth oxyca rbonyl-5-(p-tolu oyl-[1,2,3]-triazol-1-yl)-β-D-ribofuranose |
| 28 | 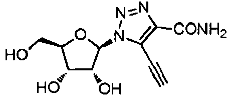 | 1'-(4-carbamoyl-5-éthynyl-[1,2,3]-triazol-1-yl)-β-D-ribofuranose |
| 29 | 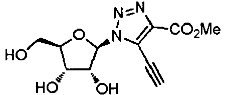 | 1'-(4-méthoxycarbonyl-5-éthynyl-[1,2,3]-triazol-1-yl)-β-D-ribofuranose |
| 30 | 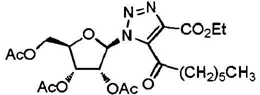 | 1'-(4-ethoxycarbonyl-5-heptanoyl-[1,2,3]-triazol-1-yl)-2',3',5'-tri-O-acétyl-β-D-ribofuranose |
| 31 | 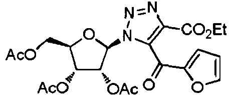 | 1'-(4-ethoxycarbony)-5-(2-furoyl)-[1,2,3]-triazol-1-yl)-2',3',5'-tri-O-acétyl-β-D-ribofuranose |
| 32 | 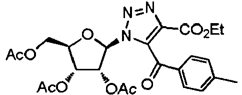 | 1'-(4-ethoxycarbonyl-5-p-toluoyl-[1,2,3]-triazol-1-yl)-2',3',5'-tri-O-acétyl-β-D-ribofuranose |

### a. Mesure de la viabilité cellulaire de 13 composés selon l'invention :

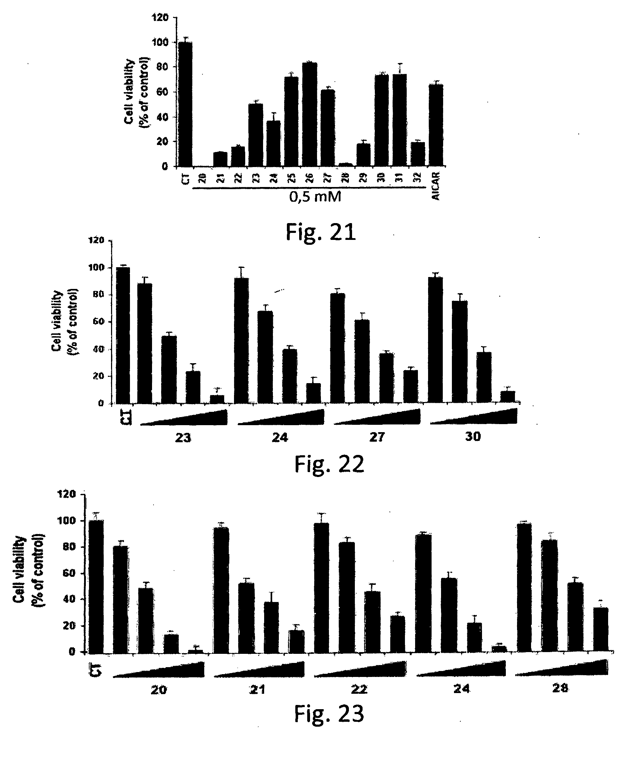

Afin de cribler les 13 composés selon l'invention, l'activité métabolique des cellules K562 est étudiée après 48 heures de traitement par 0,5 mM (dose efficace d'AICAR) de ces différents composés. La figure 21 représente le pourcentage de cellules K562 sensibles à l'Imatinib (K562) viables après stimulation ou non pendant 48 heures avec de l'AICAR ou l'un des 13 composés selon l'invention (0,5 mM) décrits dans le tableau 1 ci-dessus en plaque 96 puits. Après ajout de réactif XTT, l'absorbance est mesurée à 490nm.

Les résultats obtenus montrent que, parmi ces 13 composés, 13 composés induisent, après 48 heures de stimulation une diminution de la viabilité cellulaire. Cette diminution de la viabilité cellulaire est plus importante que le témoin positif. Elle est également plus importante ou comparable au composé AICAR. Ainsi, ces 13 composés apparaissent être des candidats intéressants comme principes actifs destinés au traitement de la LMC.

En outre, parmi ces 13 composés, 6 composés (20, 21, 22, 28, 29 et 32) induisent, après 48 heures de stimulation, une importante diminution de la viabilité cellulaire. En effet, le pourcentage de cellules viables passe dès 48 heures de traitement à des valeurs inférieures à 20%.

### b. Expériences de dose réponse pour 15 composés selon l'invention :

Les figures 22 et 23 représentent des expériences dose-réponse sur des cellules K562 exposées pendant 48 heures à des doses croissantes (5, 10, 25 et 50 µM) d'un des 9 composés choisis parmi les composés 23, 24, 27, 30, 20, 21, 22, 24 et 25.
Les histogrammes des figures 22 et 23 illustrent l'effet dose-réponse de chacun de ces 9 composés. Après ajout de réactif XTT, l'absorbance est mesurée à 490 nm.

Les résultats illustrés aux figures 22 et 23 montrent une diminution dose-dépendante du nombre de cellules viables pour les 9 composés dès 48 heures de stimulation. Ces 9 composés se révèlent tous particulièrement efficaces sur les cellules K562 à des concentrations de 50 µM. L'IC50 est de l'ordre de 5 µM pour les composés 20, 21, 23, 24 et 30, et d'environ 10 µM pour les composés 22, 27, 28 et 61.

### Exemple 3: Mode opératoire concernant la synthèse de composés selon l'invention.

A une solution de 1',2',3',5'-tétraacétate-β-D-ribose dans 50 ml de dichlorométhane sont ajoutés l'azoture de triméthylsilyle puis le BF₃-Et₂O goutte-à-goutte. Le milieu réactionnel est laissé à température ambiante pendant environ 1h puis est lavé avec une solution aqueuse saturée d'hydrogénocarbonate de sodium. La phase organique est séchée sur sulfate de magnésium puis le solvant est évaporé sous pression réduite. L'huile obtenue est purifiée sur colonne de silice. On obtient le composé 1 sous forme d'une huile incolore :

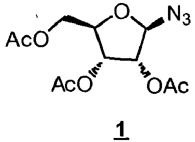

A une solution de 4-methylbenzyl bromide dans 50 ml de dimethyl-formamide est ajouté l'azoture de sodium. Le milieu réactionnel est laissé à température ambiante pendant environ 2h, le solvant est évaporé sous pression réduite et 100 ml de dichlorométhane sont ajoutés. La phase organique est lavée 2 fois à l'eau puis avec une solution saturée de NaCl, séchée sur sulfate de magnésium puis le solvant est évaporé sous pression réduite. L'huile obtenue est purifiée sur colonne de silice. On obtient le composé 2 sous forme d'une huile incolore :

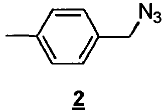

A une solution de l'azide 1 dans 5 ml de tetrahydrofurane (ou le 2-méthyl-tetrahydrofurane) placée à 0°C sont ajoutés successivement l'éthyle propiolate, le cyanure de cuivre (I), l'eau oxygénée à 35% et la N,N-Diisopropylethylamine. Le milieu réactionnel est laissé sous agitation à 0°C pendant environ 10 minutes puis ramené à température ambiante. Après la fin de la réaction, le mélange réactionnel est filtré sur lit de célite et le solvant est évaporé sous pression réduite. Le brut obtenu est purifié sur colonne de silice. On obtient le composé 3 sous forme d'une huile brune. Dans le procédé optimisé le 2-méthyl-tetrahydrofurane et le bromo-tris-(triphenylphosphine) CuBr(PPh₃)₃ sont utilisés.

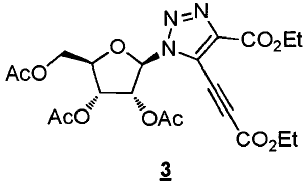

A une solution de l'azide 2 dans 5 ml de tetrahydrofurane placée à 0°C sont ajoutés successivement l'éthyle propiolate, le cyanure de cuivre (I), l'eau oxygénée à 35% et la N,N-Diisopropylethylamine. Le milieu réactionnel est laissé sous agitation à 0°C pendant environ 10 minutes puis ramené à température ambiante. Après la fin de la réaction, le mélange réactionnel est filtré sur lit de célite et le solvant est évaporé sous pression réduite. Le brut obtenu est purifié sur colonne de silice. On obtient le composé 4 sous forme d'un solide jaune. Dans le procédé optimisé le 2-méthyl-tetrahydrofurane et le bromo-tris-(triphenylphosphine) CuBr(PPh₃)₃ sont utilisés.

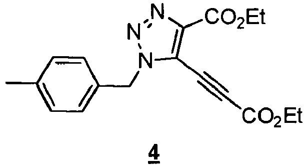

A une solution de l'azide 1 dans 20 ml de tétrahydrofurane (ou le 2-méthyl-tetrahydrofurane) le sont ajoutés l'éthyle propiolate, I2, le cérium ammonium nitrate, l'iodure de cuivre (I) et la N,N-Diisopropylethylamine. Le milieu réactionnel est laissé à température ambiante pendant environ 15 minutes puis est filtré sur lit de célite. Le solvant est évaporé sous pression réduite et l'huile sombre obtenue est purifiée sur colonne de silice. On obtient le composé 5 sous forme de solide brun. Dans le procédé optimisé le 2-méthyl-tetrahydrofurane est utilisé au lieu du THF.

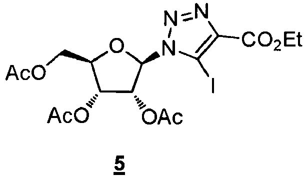

A une solution de l'azide 1 dans 10 ml de dichlorométhane sont ajoutés l'éthyle propiolate, la N-chlorosuccinimide, le chlorure de cuivre (I) et la N,N'-diisopropyléthylamine. Le milieu réactionnel est laissé à température ambiante pendant 2 heures puis est filtré sur lit de célite. Le solvant est évaporé sous pression réduite et l'huile obtenue est purifiée sur colonne de silice. On obtient le composé 6 sous forme d'huile.

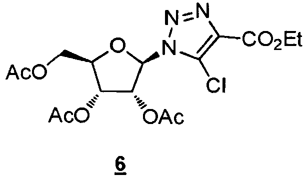

A une solution de l'azide 1 dans 5 ml de tétrahydrofurane (ou le 2-méthyl-tetrahydrofurane) sont ajoutés l'éthyle propiolate, le chlorure de p-toluoyle, l'iodure de cuivre (I) et la N,N'-diisopropyléthylamine. Le milieu réactionnel est laissé à température ambiante pendant 30 minutes puis est filtré sur lit de célite. Le solvant est évaporé sous pression réduite et l'huile obtenue est purifiée sur colonne de silice. On obtient le composé 7 sous forme d'huile.

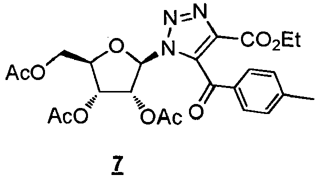

A une solution de 5 dans 20 mL de toluène sont ajoutés l'éthynyltriéthylsilane, le Pd(PPh3)2Cl2, l'iodure de cuivre (I) et la triéthylamine. Le milieu réactionnel est chauffé à 60°C pendant 90 minutes puis est filtré sur lit de célite et le solvant est évaporé sous pression réduite. L'huile obtenue est purifiée sur colonne de silice. On obtient le composé 8 sous forme d'huile.

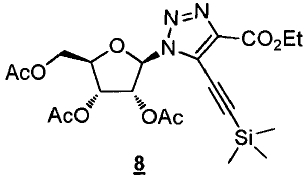

Une solution de 8 dans 40 mL de méthanol est placée dans un bain d'eau glacée. On fait buller de l'ammoniac pendant quelques secondes puis on laisse revenir le milieu réactionnel à température ambiante. Après une nuit, le solvant est évaporé sous pression réduite et l'huile obtenue est purifiée sur colonne de silice. On obtient les composés 9 et 10 sous forme de mousses.

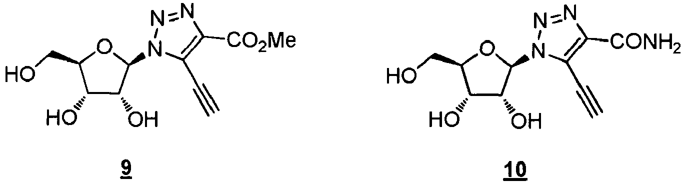

A une solution de 5 dans 10 ml de toluène sont ajoutés le 2 (tributylstannyl)thiophène, le Pd(PPh₃)₂Cl₂, l'iodure de cuivre (I) et la triéthylamine . Le milieu réactionnel est chauffé à 80°C pendant environ 3h, filtré sur lit de célite puis le solvant est évaporé sous pression réduite. L'huile obtenue est purifiée sur colonne de silice. On obtient le composé 11 sous forme d'huile brune :

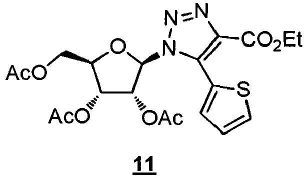

A une solution de 11 dans 20 ml d'éthanol est ajouté le carbonate de sodium. Le milieu réactionnel est laissé sous agitation à température ambiante pendant une heure, puis le solvant est évaporé sous pression réduite et le brut obtenu est purifié sur colonne de silice. On obtient le composé 12 sous forme d'une huile jaune :

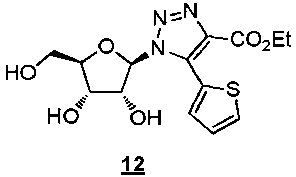

Une solution de 11 dans 30 ml de méthanol est placée dans un bain d'eau glacée. On fait barboter de l'ammoniac gazeux pendant quelques secondes puis on laisse revenir le milieu réactionnel à température ambiante. Après une nuit, le solvant est évaporé sous pression réduite et l'huile obtenue est purifiée sur colonne de silice.

On obtient le composé 13 sous forme d'une huile jaune :

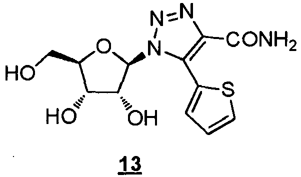

### Exemple 4: Mode opératoire concernant la synthèse de composés selon l'invention.

A une solution d'azide 1 (1-azido-2,3',4'-tri-O-acetyl Ribose) dans 20 ml de méthanol est ajouté le carbonate de sodium. Le milieu réactionnel est laissé sous agitation à température ambiante pendant une heure, puis le solvant est évaporé sous pression réduite et le brut obtenu est purifié sur colonne de silice. On obtient le composé 14 sous forme d'une huile incolore :

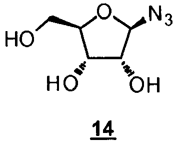

A une solution de l'azide 14 dans 20 ml d'acétone placée à 0°C sont ajoutés successivement le 2,2-diméthoxypropane puis l'éthérate de trifluorure de bore goutte à goutte. Le mélange est laissé sous agitation à 0°C pendant 30 minutes puis à température ambiante pendant une heure. Le mélange réactionnel est versé dans une solution de triéthylamine (10%) dans 20 ml d'acétone, placée à 0°C. Après 10 minutes sous agitation, la solution neutralisée est évaporée et l'huile jaune obtenue est purifiée sur colonne de silice (éluant CH2CI2-MeOH : 98-2). On obtient le composé 15 sous forme d'huile incolore :

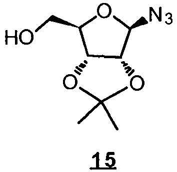

A une solution de l'azide 15 dans 5 ml de tetrahydrofurane (ou le 2-méthyl-tetrahydrofurane) lacée à 0°C sont ajoutés successivement l'éthyle propiolate, le cyanure de cuivre (I), l'eau oxygénée à 35% et la N,N-Diisopropylethylamine. Le milieu réactionnel est laissé sous agitation à 0°C pendant environ 10 minutes puis ramené à température ambiante. Après la fin de la réaction, le mélange réactionnel est filtré sur lit de célite et le solvant est évaporé sous pression réduite. Le brut obtenu est purifié sur colonne de silice (éluant : cyclohexane-AcOEt : 7-3). On obtient le composé 16 sous forme d'une huile. Dans le procédé optimisé le 2-méthyl-tetrahydrofurane et le bromo-tris-(triphenylphosphine) CuBr(PPh₃)₃ sont utilisés.

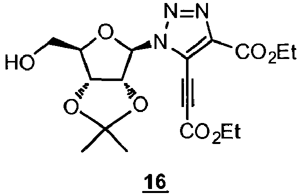

A une solution de 16 dans 2 ml de l'anhydride acétique est ajouté le 4-Dimethylaminopyridine (DMAP). Le milieu réactionnel est laissé sous agitation à température ambiante pendant deux heures. Après la fin de la réaction, le solvant est évaporé sous pression réduite. Le brut obtenu est purifié sur colonne de silice (éluant : cyclohexane-AcOEt : 9-1). On obtient le composé 17 sous forme d'une huile:

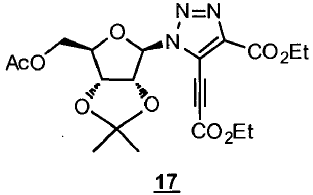

A une solution de l'azide 15 dans 20 ml de tétrahydrofurane (ou le 2-méthyl-tetrahydrofurane) sont ajoutés l'éthyle propiolate, I₂, le cérium ammonium nitrate, l'iodure de cuivre (I) et la N,N-Diisopropylethylamine. Le milieu réactionnel est laissé à température ambiante pendant environ 20 minutes puis est filtré sur lit de célite. Le solvant est évaporé sous pression réduite et l'huile sombre obtenue est purifiée sur colonne de silice (éluant : cyclohexane-AcOEt : 8-2). On obtient le composé 18 sous forme de solide brun : 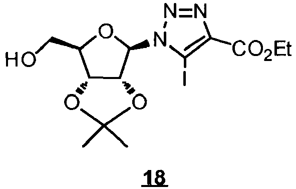
a- (couplage de Stille) : A une solution de 18 dans 10 ml de toluène sont ajoutés le 2-(tributylstannyl)thiophène, le Pd(PPh₃)₄ et l'iodure de cuivre (I). Le milieu réactionnel est chauffé à 80°C pendant environ 2h, filtré sur lit de célite puis le solvant est évaporé sous pression réduite. L'huile obtenue est purifiée sur colonne de silice (éluant : cyclohexane-AcOEt : 8-2). On obtient le composé 19 sous forme d'huile.
b- (couplage de Suzuki): A une solution du composé 18 dans le dioxane (2 ml), on ajoute 0,5 ml d'une solution de Na₂CO₃ (1M) puis l'acide thiophène-2-boronique. Le mélange est ensuite dégazé et mis sous argon puis on ajoute le catalyseur Pd(PPh₃)₄. On chauffe à 85°C sous agitation pendant 4h. On neutralise le mélange réactionnel avec 10 ml de HCl (1M) et on ajoute 20 ml d'acétate d'éthyle. La phase organique est ensuite extraite avec une solution de NaCl et évaporée sous pression réduite. Le résidu brut est purifié sur colonne de silice (éluant : cyclohexane-AcOEt : 8-2). On obtient le composé 19 sous forme d'huile :

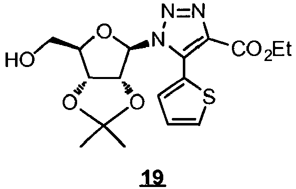

A une solution de 19 dans 2 ml de l'anhydride acétique est ajouté le 4-Dimethylaminopyridine (DMAP). Le milieu réactionnel est laissé sous agitation à température ambiante pendant deux heures. Après la fin de la réaction, le solvant est évaporé sous pression réduite. Le brut obtenu est purifié sur colonne de silice (éluant : cyclohexane-AcOEt : 9-1). On obtient le composé 20 sous forme d'une huile:

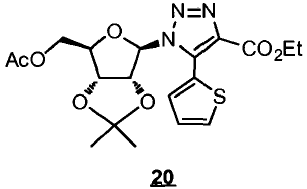

A une solution de 1'-acétyl deoxyribose dans 60 ml de pyridine est ajouté le chlorure de toluoyle. Le mélange réactionnel est laissé à température ambiante pendant 30 minutes. Après extraction au dichlorométhane, séchage sur sulfate de magnésium et filtration, le solvant est évaporé sous pression réduite et l'huile obtenue est purifiée sur colonne de silice (éluant : cyclohexane-AcOEt : 8-2). On obtient le composé 21 sous forme d'une huile jaune qui se solidifie pour donner un solide beige :

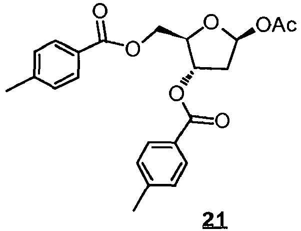

A une solution de 21 dans 50 ml de dichlorométhane sont ajoutés l'azoture de triméthylsilyle puis le BF₃-Et₂O goutte-à-goutte. Le milieu réactionnel est laissé à température ambiante pendant environ 3h puis est lavé avec une solution aqueuse saturée d'hydrogénocarbonate de sodium. La phase organique est séchée sur sulfate de magnésium puis le solvant est évaporé sous pression réduite. L'huile obtenue est purifiée sur colonne de silice (éluant : cyclohexane-AcOEt : 9-1). On obtient le composé 22 sous forme d'une huile incolore :

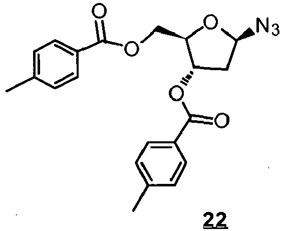

A une solution de l'azide 22 dans 5 ml de tetrahydrofurane placée à 0°C sont ajoutés successivement l'éthyle propiolate, le cyanure de cuivre (I), l'eau oxygénée à 35% et la N,N-Diisopropylethylamine. Le milieu réactionnel est laissé sous agitation à 0°C pendant environ 10 minutes puis ramené à température ambiante. Après la fin de la réaction, le mélange réactionnel est filtré sur lit de célite et le solvant est évaporé sous pression réduite. Le brut obtenu est purifié sur colonne de silice (éluant : cyclohexane-AcOEt : 8-2). On obtient le composé 23 sous forme d'un solide. Dans le procédé optimisé le 2-méthyl-tetrahydrofurane et le bromo-tris-(triphenylphosphine) CuBr(PPh₃)₃ sont utilisés.

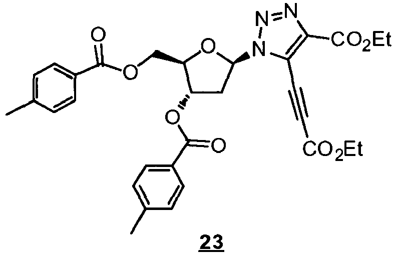

A une solution de 1,2,3,5-tétra-O-benzoyi-β-L-ribofuranose dans 100 ml de dichlorométhane sont ajoutés l'azoture de triméthylsilyle puis le trifluorure borane éthérate goutte-à-goutte. Le milieu réactionnel est laissé à température ambiante pendant 2 heures puis est lavé avec une solution aqueuse saturée d'hydrogénocarbonate de sodium. La phase organique est séchée sur sulfate de magnésium, filtrée puis le solvant est évaporé sous pression réduite. L'huile obtenue est purifiée sur colonne de silice (éluant : cyclohexane-AcOEt : 9-1). On obtient le composé 24 sous forme d'un solide blanc.

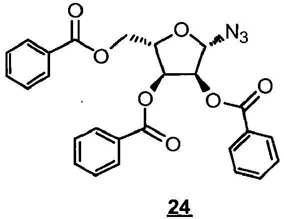

A une solution de l'azide 24 dans 5 ml de tetrahydrofurane placée à 0°C sont ajoutés successivement l'éthyle propiolate, le cyanure de cuivre (I), l'eau oxygénée à 35% et la N,N-Diisopropylethylamine. Le milieu réactionnel est laissé sous agitation à 0°C pendant environ 10 minutes puis ramené à température ambiante. Après la fin de la réaction, le mélange réactionnel est filtré sur lit de célite et le solvant est évaporé sous pression réduite. Le brut obtenu est purifié sur colonne de silice (éluant : cyclohexane-AcOEt : 8-2). On obtient le composé 25 sous forme d'un solide brun. Dans le procédé optimisé le 2-méthyl-tetrahydrofurane et le bromo-tris-(triphenylphosphine) CuBr(PPh₃)₃ sont utilisés.

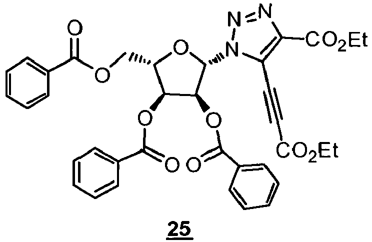

### Exemple 5: Analyse de l'efficacité de cinq composés selon l'invention sur différentes lignées hématopoïétiques et de cancers solides :

L'efficacité et les DL50 (doses létales médianes) de cinq composés selon l'invention, repris dans le tableau 3 ci-dessous ont été déterminées sur 6 lignées cellulaires d'hémopathies malignes.

**Tableau 3 :**

| Code molécule | Formule chimique de la molécule | Nom chimique de la molécule |
|---|---|---|
| 82 | 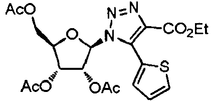 | 1'-(4-ethoxycarbonyl-5-(2-thienyl)-[1,2,3]-triazol-1-yl)-2',3',5'-tri-O-acétyl-β-D-ribofuranose |
| 86 | 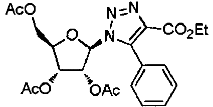 | 1'-(4-ethoxycarbonyl-5-phényl-[1,2,3]-triazol-1-yl)-2',3',5'-tri-O-acétyl-β-D-ribofuranose |
| 112 | 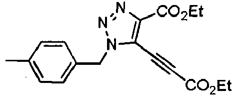 | 1-(4-méthylbenzyl)-4-ethoxycarbonyl-5-éthylpropiolate-1,2,3-triazole |
| 196 | 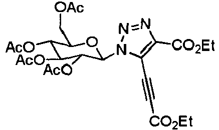 | 1'-(4-ethoxycarbonyl-5-éthylpropiolate-[1,2,3]-triazol-1-yl)-2',3',4',6'-tétra-O-acétyl-β- D-glucopyranose |
| 236 | 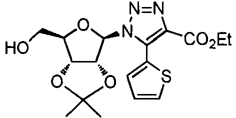 | 1'-(4-ethoxycarbony)-5-(2-thienyl)-[1,2,3]-triazol-1-yl)-2',3'-O-isopropylidène-β-D-ribofuranose |

Les résultats de cette étude sont repris dans le tableau 4 ci-dessous.

**Tableau 4 :**

| Lignées | Composé 49 | Composé 82 | Composé 86 | Composé 112 | Composé 196 | Composé 236 |
|---|---|---|---|---|---|---|
| MEC-1 (Leucémie Lymphoblastique Chronique B) | 0,2 | 0,75 | 2,5 | 1 | 0,1 | 1 |
| J16 (Lymphome T) | 2,5 | 1,5 | 5 | 2,5 | 0,75 | 2 |
| 8866 (Leucémie Lymphoblastique B) | 3 | 1,5 | 4 | 1,5 | 3 | 2,5 |
| U 937 (Lymphome Monocytaire) | 0,5 | 1 | 2,5 | 2 | 0,1 | 2 |
| RAJI (Burkitt) | >10 | 2,5 | 10 | 10 | >10 | 4 |
| 8226 (Myélome) | 1,5 | 2,5 | 2,5 | 1,5 | 0,5 | 1,5 |

De même, l'efficacité et les DL50 (doses létales médianes) de ces cinq composés repris dans le tableau 3 ci-dessus ont été déterminées sur neuf lignées cellulaires de cancers solides d'origines différentes. Les résultats de cette étude sont repris dans le tableau 5 ci-dessous.

**Tableau 5 :**

| Lignées | Composé 49 | Composé 82 | Composé 86 | Composé 112 | Composé 196 | Composé 236 |
|---|---|---|---|---|---|---|
| HCT8R (cancer du colon) | >10 | 3,5 | >10 | 10 | >10 | 4 |
| MDAM 453 (Cancer du sein) | >10 | 1 | 5 | >10 | >10 | 1 |
| A 375 (Mélanome) | 7,5 | 2 | 5 | 7,5 | 10 | 2 |
| SHSY-5Y (Neuroblastome) | >10 | 1,5 | 3 | >10 | >10 | 0,5 |
| Pc3 (Cancer de la prostate) | >10 | 3 | 10 | >10 | >10 | 5 |
| NCIH 226 (Cancer du poumon) | >10 | 2,5 | 7,5 | 10 | >10 | 4 |
| MEWO (Mélanome, p53 muté) | >10 | 1,5 | 7,5 | >10 | >10 | 3 |
| SF 278 (Glioblastome) | 10 | 1,5 | 2,5 | 10 | >10 | 2,5 |
| HEK (Cancer du rein) | >10 | 1,5 | 6 | >10 | >10 | 2,5 |

Ainsi, les résultats repris dans le tableau 4 ci-dessus montrent que toutes les molécules testées présentent une efficacité sur les hémopathies malignes. Les dérivés les plus efficaces sont les molécules 82, 196 et 236.

Dans le tableau 5 il a été constaté que les molécules sont globalement plus efficaces sur les hémopathies malignes que sur les cancers solides. Toutefois les dérivés 82 et 236 de structure très proche montrent tout de même une efficacité significative sur la totalité des cancers testés.

### Exemple 6 : Analyse « in vivo » de l'efficacité des dérivés 86, 196 et 236 :

Les trois composés (86, 196 et 236) ont été testés à 1mg/kg sur la prise tumorale de cellules de LMC K562 résistantes à l'imatinib dans des souris athymiques Nude. Les tumeurs des souris de chaque groupe sont pesées au jour du sacrifice, la somme de ces masses constitue un indice de croissance tumorale. Cet indice est exprimé en pourcentage par rapport à la croissance du groupe contrôle (seconde ligne).

Les résultats de cette analyse « in vivo » sont repris dans le tableau 6 ci-dessous.

**Tableau 6 :**

| | Contrôle | Composé 86 | Composé 196 | Composé 236 |
|---|---|---|---|---|
| Masse des tumeurs à la fin de la campagne | 100 | 69,6 | 42 | 60,3 |
| (% vs contrôle) | | | | |
| Poids moyen des souris à la date du sacrifice (g) | 18,63 | 18,43 | 20,5 | 20,14 |

Comme le montre le tableau 6 ci-dessus, les molécules ont toutes montré une efficacité sur le développement de la masse tumorale. On peut tout de même noter que le composé 196 semble être le plus efficace avec une inhibition d'environ 60% de la masse tumorale, ensuite vient le composé 236 et enfin le 86.

On note également que les souris traitées ne présentent pas de signe d'altération de leur état général suite aux traitements reçus. Un indice de l'état satisfaisant des souris est la moyenne du poids de chaque groupe présentée à la troisième ligne du tableau.

## Revendications

1. Composés de formule générale : 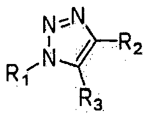 dans laquelle :
- R₁ est choisi parmi
• un groupement pentose cyclique sous forme furanique avec les groupements OH libres ou éventuellement substitués par un ou plusieurs groupements mono-, bi- ou triphosphate, acétyle, isopropylidène, benzoyle ou para-toluoyle,
• un groupement hexose sous forme pyranique avec les groupements OH libres ou éventuellement substitués par un ou plusieurs groupements mono-, bi- ou triphosphate ou acétyle,
• un groupement naphtyle, éventuellement substitué par un ou plusieurs groupements alkyles ou amines substituées ayant de 1 à 4 atomes de carbone,
• un groupement benzyle éventuellement substitué par un ou plusieurs groupements alkyles ou amines substituées ayant de 1 à 4 atomes de carbone,
• des groupes phényles, biphényles, hétéroaryles ;
- R₂ est choisi parmi
• un groupement amide -CONH₂, -CONHMe, -CONHEt, -CON(Me)₂, -CON(Et)₂,
• un groupement acide ou ester -CO₂H, CO₂Me, CO₂Et un groupement cyano ou amidine -CN, -C(NH₂)NH, -C(NHMe)NH, -C(NHEt)NH,
• un groupement phényle éventuellement substitué par un halogène choisi parmi CI, Br, I et F,
• un groupement thiophène,
• une chaine carbonée linéaire ou ramifiée ayant de 3 à 10 atomes de carbone, ou
• un groupement méthoxynaphtalène ; et
- R₃ est choisi parmi :
• un groupement halogène,
• un groupement furane ou -CO-furane,
• un groupement thiophène ou -CO-thiophène ou -C≡C-thiophène,
• un groupement toluoyle,
• un groupement acétylène,
• un groupement -CO-(CH₂)ₙ-CH₃, avec n compris entre 2 et 9,
• un groupement phényle ou -C≡C-phényl, éventuellement substitué par un halogène,
• un groupement -C≡C-CO₂Me, -C≡C-CO₂Et, -C≡C-CONH₂,
• un groupement -C≡C-(CH₂)₆CH₃, ou
• un groupement -C=C-2-méthoxynaphtalène ;
leurs racémiques, énantiomères, diastéréoisomères et leurs mélanges, leurs tautomères et leurs sels pharmaceutiquement acceptables,
pour leur utilisation comme médicament.

2. Composés selon la revendication 1, dans lesquels :
- R₁ est choisi parmi 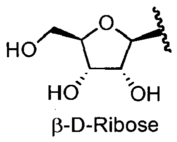 ou 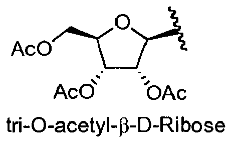 ou 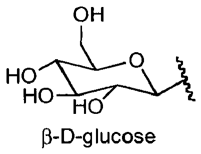 ou 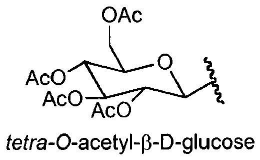 ou 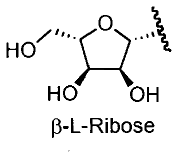 ou 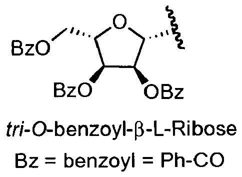 ou 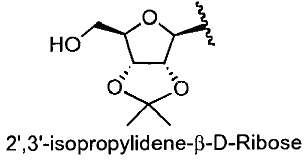 ou 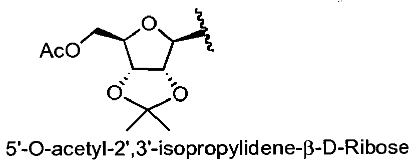 ou 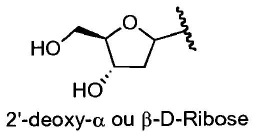 ou 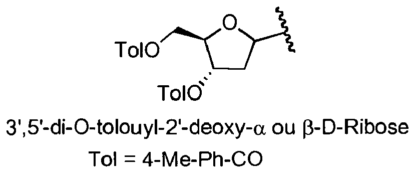 ou 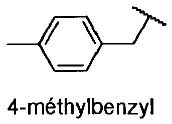 ou 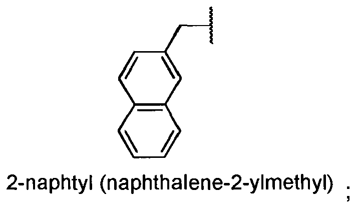
- R₂ est choisi parmi -CONH₂, -CO₂Me, -CO₂Et, phényle, thiophène, -(CH₂)₆CH₃, p-fluoro-phényl ou le 2-méthoxynaphtalène ; et
- R₃ est choisi parmi I, CI, furane, CO-furane, CO-thiophène, acétylène, CO-(CH₂)₅-CH₃, toluoyle, -C≡C-CO₂Et, thiophène, phényl, -C≡C-phényl, -C=C-thiophène, -C≡C-(CH₂)₆CH₃, -C≡C-(p-fluoro-phényl), ou -C≡C-2-méthoxynaphtalène.

3. Composés selon la revendication 2, dans lesquels R₁ est 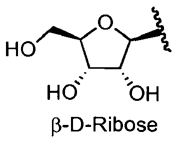 ou 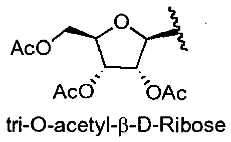 ou 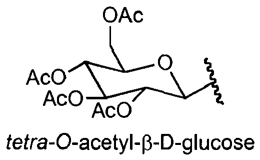 ou 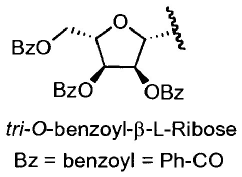 ou 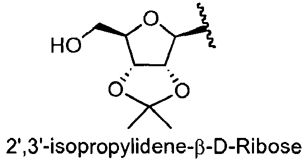 ou 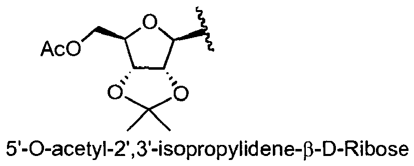 ou 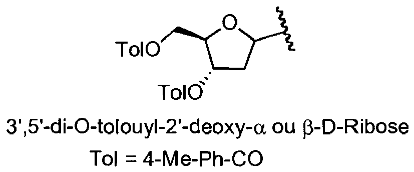 ou 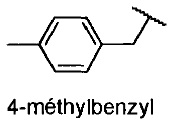 ou 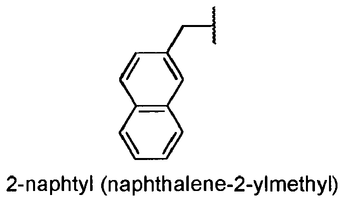 et
- lorsque R1 est un groupement β-D-ribose, alors :
- R2= CONH₂ et R3 = I, CI, CO-Furane, CO-thiophène, toluoyle ou acétylène ;
ou
- R2=CO₂Me et R3 = I, furane ou acétylène ;
ou
- R2 = phényl et R3 = I;
- lorsque R1 est un groupement tri-O-acétyt-β-D-ribose, alors :
- R2=CO₂Et et R3 = CI, CO-(CH₂)₅-CH₃, CO-furane, toluoyle, -C≡C-CO₂Et, thiophène ou phényl ;
ou
- R2= phényl et R3 = -C=C-phényl ;
ou
- R2 = thiophène et R3 = -C≡C-thiophène ;
ou
- R2 = (CH₂)₆CH₃ et R3 = -C≡C-(CH₂)₆CH₃ ;
ou
- R2 = p-fluoro-phényl et R3 = -C=C-p-fluoro-phényl ;
ou
- R2 = 2-methoxynaphtalene et
R3 = -C=C-2-methoxynaphtalene ;
- lorsque R1 est un groupement tétra-O-acétyl-β-D-glucose, alors R2= CO₂Et et R3 = I ou -C≡C-CO₂Et ;
- lorsque R1 est un groupement tri-O-benzoyl-β-L-ribose, alors R2= CO₂Et et R3= -C≡C-CO₂Et ;
- lorsque R1 est un groupement 2',3'-isopropylidene-β-D-ribose, alors R2 = CO₂Et et R3= -C≡C-CO₂Et ou thiophène ;
- lorsque R1 = 5'-O-acetyl-2',3'-isopropylidene-β-D-ribose, alors R2 = CO₂Et et R3= -C≡C-CO₂Et ou thiophène ;
- lorsque R1 est un groupement 3',5'-di-O-tolouyl-2'-deoxy-β-D-ribose, alors R2= CO₂Et et R3= -C≡C-CO₂Et ;
- lorsque R1 est un groupement 4-méthylbenzyl, alors :
- R2= CO₂Et et R3 = -C≡C-CO₂Et ;
ou
- R2= phényl et R3 = -C≡C-phényl ;
- lorsque R1 est un groupement 2-naphtyl (naphthalene-2-ylmethyl), alors :
- R2= CO₂Et et R3 = I ;
ou
- R2= CO₂Et et R3 = -C≡C-CO₂Et ;
ou
- R2= Phényl et R3 = -C≡C-phényl.

4. Composés selon la revendication 3, dans lesquels R1 est 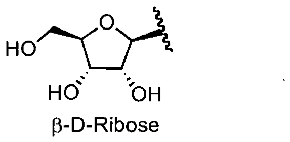 ou 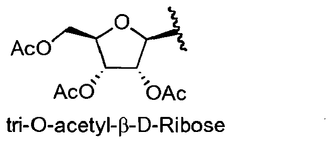 ou 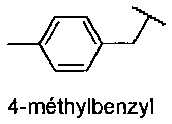 ou 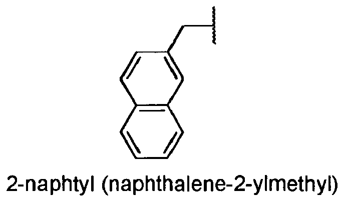 et
- lorsque R1 est un groupement β-D-ribose, alors :
- R2= CONH₂ et R3 = Cl, CO-furane, CO-thiophène ou toluoyle ;
ou
- R2= CO₂Me et R3 = I ou acétylène ;
ou
- R2 = phényl et R3 = I ;
- lorsque R1 est un groupement tri-O-acétyl-β-D-ribose, alors :
- R2= CO₂Et et R3 = CO-(CH₂)₅-CH₃, CO-furane, toluoyle, -C≡C-CO₂Et, thiophène ou phényl ;
ou
- R2= phényl et R3 = -C≡C-phényl ;
ou
- R2 = thiophène et R3 = -C≡C-thiophène ;
ou
- R2 = (CH₂)₆CH₃ et R3 = -C≡C-(CH₂)₆CH₃ ;
ou
- R2 = p-fluoro-phényl et R3 = -C=C-p-fluoro-phényl ;
ou
- R2 = 2-methoxynaphtalene et
R3 = -C≡C-2-methoxynaphtalene ;
- lorsque R1 est un groupement 4-méthylbenzyl, alors :
- R2= CO₂Et et R3 = -C≡C-CO₂Et ;
ou
- R2= phényl et R3 = -C≡C-phényl ;
- lorsque R1 est un groupement 2-naphtyl (naphthalene-2-yl-methyl), alors :
- R2= CO₂Et et R3 = I ;
ou
- R2= CO₂Et et R3 = -C≡C-CO₂Et ;
ou
- R2= Phényl et R3 = -C≡C-phényl.

5. Composés selon la revendication 4, lesdits composés étant choisis parmi :
- 1'-(4-ethoxycarbonyl-5-iodo-[1,2,3]-triazol-1-yl)-2',3',5'-tri-O-acétyl-β-D-ribofuranose;
- 1'-(4-carbamoyl-5-iodo-[1,2,3]-triazol-1-yl)-β-D-ribofuranose;
- 1'-(4-méthoxycarbonyl-5-éthynyl-[1,2,3]-triazol-1-yl)-β-D-ribofuranose;
- 1-(naphtyl-2-methyl)-4-ethoxycarbonyl-5-iodo-1,2,3-triazole;
- 1-(naphtyl-2-methyl)-4-ethoxycarbonyl-5-éthylpropiolate-1,2,3-triazole;
- 1'-(4-ethoxycarbonyl-5-éthylpropiolate-[1,2,3]-triazol-1-y1)-2',3',5'-tri-O-acétyl-β-D-ribofuranose;
- 1'-(4-ethoxycarbonyl-5-(2-thienyl)-[1,2,3]-triazol-1-yl)-2',3',5'-tri-O-acétyl-β-D-ribofuranose;
- 1'-(4-ethoxycarbonyl-5-phényl-[1,2,3]-triazol-1-yl)-2',3',5'-tri-O-acétyl-β-D-ribofuranose;
- 1-(4-méthylbenzyl)-4-ethoxycarbonyl-5-éthylpropiolate-1,2,3-triazole;
- 1'-(4-heptyl-5-(non-1-yn-1-yl)-[1,2,3]-triazol-1-yl)-2',3',5'-tri-O-acétyl-β-D-ribofuranose;
- 1'-(4-ethoxycarbonyl-5-éthylpropiolate-[1,2,3]-triazol-1-yl)-2',3',5'-tri-O-benzoyl-β-L-ribofuranose;
- 2'-désoxy-1'-(4-éthoxycarbonyl-5-éthylpropiolate-[1,2,3]-triazol-1-yl)-3',5'-di-O-(p-toluoyl)-α-D-ribofuranose
- 1'-(4-ethoxycarbonyl-5-éthylpropiolate-[1,2,3]-triazol-1-yl)-2',3',4',6'-tétra-O-acétyl-β-D-glucopyranose;
- 1'-(4-ethoxycarbonyl-5-éthylpropiolate-[1,2,3]-triazol-1-yl)-2',3'-O-isopropylidène-β-D-ribofuranose;
- 1'-(4-ethoxycarbonyl-5-éthylpropiolate-[1,2,3]-triazol-1-yl)-2',3'-O-isopropylidène-5'-O-acétyl-β-D-ribofuranose;
- 1'-(4-ethoxycarbonyl-5-(2-thienyl)-[1,2,3]-triazol-1-yl)-2',3'-O-isopropylidène-β-D-ribofuranose; et
- 1'-(4-ethoxycarbonyl-5-(2-thienyl)-[1,2,3]-triazol-1-yl)-2',3'-O-isopropylidène-5'-O-acétyl-β-D-ribofuranose.

6. Composés selon la revendication 5, lesdits composés étant choisis parmi :
- 1'-(4-ethoxycarbonyl-5-(2-thienyl)-[1,2,3]-triazol-1-yl)-2',3',5'-tri-O-acétyl-β-D-ribofuranose;
- 1'-(4-ethoxycarbonyl-5-éthylpropiolate-[1,2,3]-triazol-1-yl)-2',3',5'-tri-O-acétyl-β-D-ribofuranose;
- 1'-(4-ethoxycarbonyl-5-éthylpropiolate-[1,2,3]-triazol-1-yl)-2',3'-O-isopropylidène-β-D-ribofuranose;
- 1'-(4-ethoxycarbonyl-5-éthylpropiolate-[1,2,3]-triazol-1-yl)-2',3'-O-isopropylidène-5'-O-acétyl-β-D-ribofuranose; et
- 1-(4-méthylbenzyl)-4-ethoxycarbonyl-5-éthylpropiolate-1,2,3-triazole.

7. Composés selon la revendication 5, lesdits composés étant choisis parmi :
- 1'-(4-ethoxycarbonyl-5-(2-thienyl)-[1,2,3]-triazol-1-yl)-2',3',5'-tri-O-acétyl-β-D-ribofuranose;
- 1'-(4-ethoxycarbonyl-5-phényl-[1,2,3]-triazol-1-yl)-2',3',5'-tri-O-acétyl-β-D-ribofuranose;
- 1-(4-méthylbenzyl)-4-ethoxycarbonyl-5-éthylpropiolate-1,2,3-triazole;
- 1'-(4-ethoxycarbonyl-5-éthylpropiolate-[1,2,3]-triazol-1-yl)-2',3',4',6'-tétra-O-acétyl-β-D-glucopyranose; et
- 1'-(4-ethoxycarbonyl-5-(2-thienyl)-[1,2,3]-triazol-1-yl)-2',3'-O-isopropylidène-β-D-ribofuranose.

8. Composés selon l'une des revendications 1 à 7, pour leur utilisation dans le traitement des maladies métaboliques.

9. Composés selon l'une des revendications 1 à 7, pour leur utilisation dans le traitement du cancer.

10. Composés selon la revendication 9, pour leur utilisation dans le traitement des tumeurs solides.

11. Composés selon la revendication 9, pour leur utilisation dans le traitement des hémopathies malignes.

12. Composés selon l'une des revendications 1 à 7, pour leur utilisation dans le traitement de la leucémie myéloïde chronique (LMC).

13. Composés selon la revendication 9, pour leur utilisation dans le traitement des cancers résistants aux inhibiteurs de tyrosine kinases.

14. Composés selon la revendication 12, pour leur utilisation dans le traitement des leucémies myéloïdes chroniques (LMC) résistantes aux inhibiteurs de tyrosine kinases.

15. Produit pharmaceutique contenant un composé selon l'une des revendications 1 à 7 et au moins un deuxième actif comme produit de combinaison pour une administration simultanée, séparée ou étalée dans le temps, pour son utilisation dans le traitement du cancer.

16. Composition pharmaceutique comprenant un composé selon l'une des revendications 1 à 7 et un support pharmaceutiquement acceptable.

## Patentansprüche

1. Verbindungen mit der allgemeinen Formel: 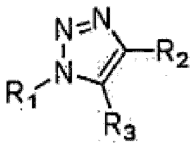 worin:
- R₁ ausgewählt ist aus
- einer cyclischen Pentosegruppe in Furanform, wobei die OH-Gruppen frei oder eventuell durch eine oder mehrere Mono-, Bi- oder Triphosphat-, Acetyl-, Isopropyldiene-, Benzoyl- oder para-Tolyol-Gruppen substituiert sind,
- einer Hexosegruppe in Pyranform, wobei die OH-Gruppen frei oder eventuell durch eine oder mehrere Mono-, Bi- oder Triphosphat- oder AcetylGruppen substituiert sind,
- einer Naphthylgruppe, eventuell substituiert durch eine oder mehrere substituierte Alkyl- oder Aminogruppen mit 1 bis 4 Kohlenstoffatomen,
- eine Benzylgruppe, eventuell substituiert durch eine oder mehrere substituierte Alkyl- oder Aminogruppen mit 1 bis 4 Kohlenstoffatomen,
- Phenyl-, Biphenyl, Heteroarylgruppen;
- R₂ ausgewählt ist aus
- einer Amidgruppe -CONH₂, -CONHMe, -CONHEt, -CON(Me)₂,-CON(Et)₂,
- einer Säure- oder Estergruppe -CO₂H, CO₂Me, CO₂Et, einer Cyano-oder Amidingruppe -CN, -C(NH₂)NH, -C(NHMe)NH, -C(NHEt)NH,
- einer Phenylgruppe, eventuell substituiert durch ein Halogen, ausgewählt aus Cl, Br, I und F,
- einer Thiophengruppe,
- einer linearen oder verzweigten Kohlenwasserstoffkette mit 3 bis 10 Kohlenstoffatomen, oder
- einer Methoxynaphtalengruppe; und
- R₃ ausgewählt ist aus:
- einer Halogengruppe,
- einer Furan- oder -CO-Furangruppe,
- einer Thiophen- oder -CO-Thiophen- oder C=C-Thiophengruppe,
- einer Toluoylgruppe,
- einer Acetylengruppe,
- einer -CO-(CH₂)ₙ-CH₃-Gruppe, wobei n zwischen 2 und 9 liegt,
- einer Phenyl- oder -C≡C-Phenylgruppe, eventuell substituiert durch ein Halogen,
- einer -C≡C-CO₂Me-, -C≡C-CO₂Et-, -C≡C-CONH₂-Gruppe,
- einer -C≡C-(CH₂)₆CH₃-Gruppe, oder
- einer -C≡C-2-methoxynaphtalen-Gruppe;
ihren Racematen, Enantiomeren, Diastereoisomeren und ihren Mischungen, ihren Tautomeren und ihre pharmazeutisch akzeptablen Salze,
für ihre Verwendung als Medikament.

2. Verbindungen nach Anspruch 1, wobei;
- R₁ ausgewählt ist aus 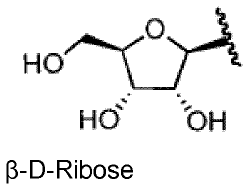 oder 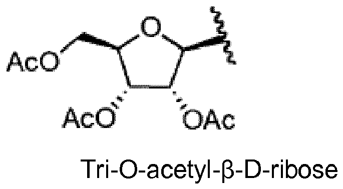 oder 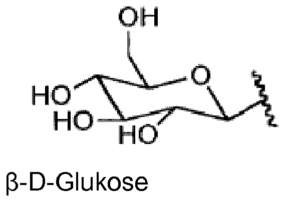 oder 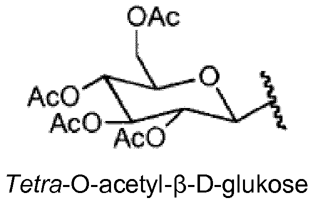 oder 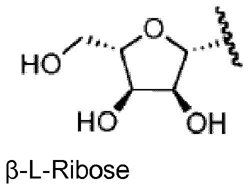 oder 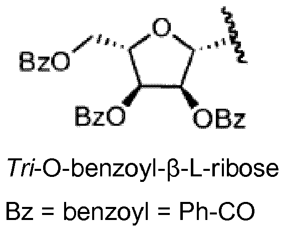 oder 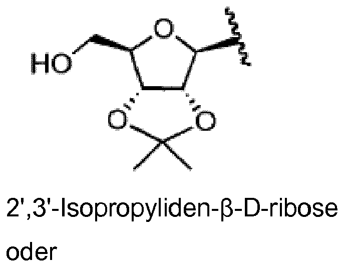 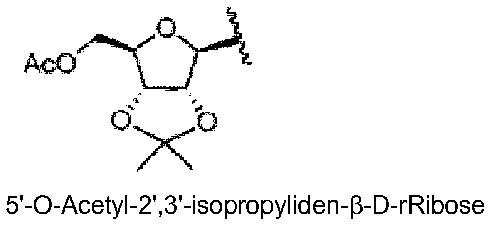 oder 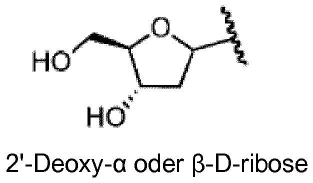 oder 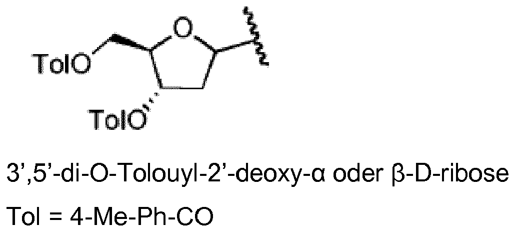 oder 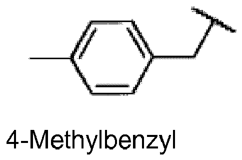 oder 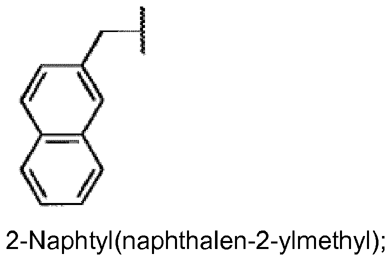
- R₂ ausgewählt ist aus -CONH₂, -CO₂Me, -CO₂Et, Phenyl, Thiophen, - (CH₂)₆CH₃, p-Fluorphenyl oder 2-Methoxynaphtalen; und
- R₃ ausgewählt ist aus I, Cl, Furan, CO-Furan, CO-Thiophen, Acetylen, CO-(CH₂)₅-CH₃, Toluoyl, -C≡C-CO₂Et, Thiophen, Phenyl, -C=C-Phenyl, -C=C-Thiophen, -C≡C-(CH₂)₆CH₃, -C=C-(p-Fluorphenyl), oder
- C≡C-2-Methoxynaphtalen.

3. Verbindungen nach Anspruch 2, wobei R₁ Folgendes ist: 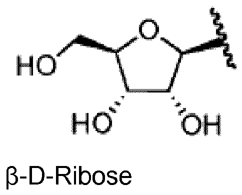 oder 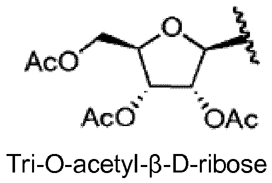 oder 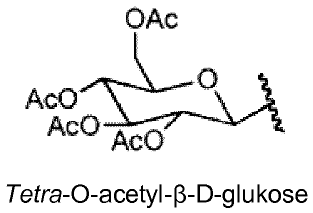 oder 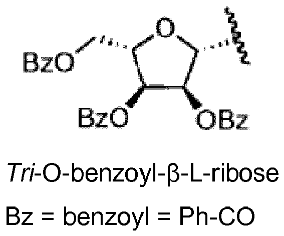 oder 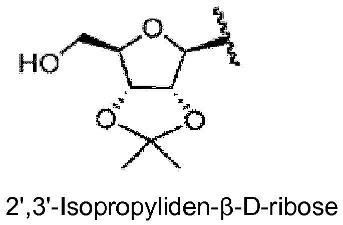 oder 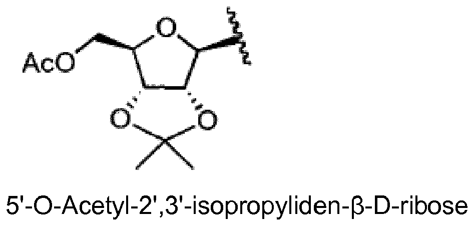 oder 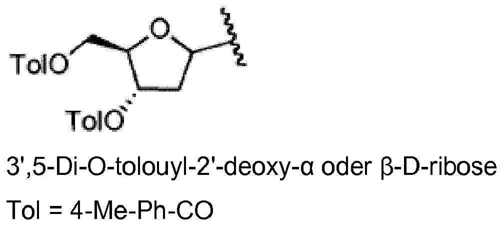 oder 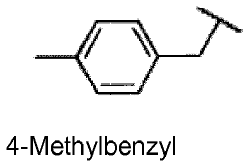 oder 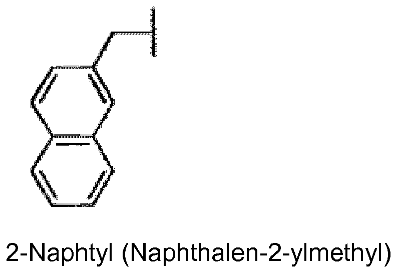 und
- wenn R1 eine β-D-Ribose-Gruppe ist, dann:
- R2 = CONH₂ und R3 = I, Cl, CO-Furan, CO-Thiophen, Toluoyl oder Acetylen;
oder
- R2 = CO₂Me und R3 = I, Furan oder Acetylen;
oder
- R2 = Phenyl und R3 = I;
- wenn R1 eine Tri-O-acetyl-β-D-ribose-Gruppe ist, dann:
- R2 = CO₂Et und R3 = Cl, CO-(CH₂)₅-CH₃, CO-Furan, Toluoyle, -C=C-CO₂Et, Thiophen oder Phenyl;
oder
- R2 = Phenyl und R3 = -C≡C-Phenyl;
oder
R2 = Thiophen und R3 = -C=C-Thiophen;
oder
- R2 = (CH₂)₆CH₃ und R3 = -C≡C-(CH₂)₆CH₃;
oder
- R2 = p-Fluorphenyl und R3 = -C≡C-p-Fluorphenyl;
oder
- R2 = 2-Methoxynaphtalen und R3 = -C≡C-2-Methoxynaphtalen;
- wenn R1 eine Tetra-O-acetyl-β-D-glukose-Gruppe ist, dann R2 = CO₂Et und R3 = I oder -C≡C-CO₂Et;
- wenn R1 eine Tri-O-benzoyl-β-L-ribose-Gruppe ist, dann R2 = CO₂Et und R3= -C≡C-CO₂Et;
- wenn R1 eine 2',3'-isopropylidene-β-D-ribose-Gruppe ist, dann R2 = CO₂Et et R3 = -C≡C-CO₂Et oder Thiophen;
- wenn R1 = 5'-O-acetyl-2',3'-isopropylidene-β-D-ribose, dann R2 = CO₂Et und R₃ = -C≡C-CO₂Et oder Thiophen;
- wenn R1 ein 3',5'-Di-O-tolouyl-2'-deoxy-β-D-ribose-Gruppe ist, dann R2 = CO₂Et und R3 = -C≡C-CO₂Et;
- wenn R1 eine 4-Methylbenzyl-Gruppe ist, dann:
- R2 = CO₂Et und R3 = -C≡C-CO₂Et;
oder
- R2 = Phenyl und R3 = -C≡C-Phenyl;
- wenn R1 eine 2-Naphtyl (Naphthalen-2-ylmethyl)-Gruppe ist, dann:
- R2 = CO₂Et und R3 = I;
oder
- R2 = CO₂Et und R3 = -C≡C-CO₂Et;
oder
R2 = Phenyl und R3 = -C≡C-phenyl.

4. Verbindungen nach Anspruch 3, wobei R1 Folgendes ist: 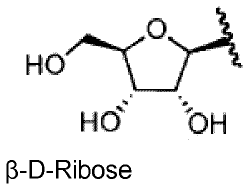 oder 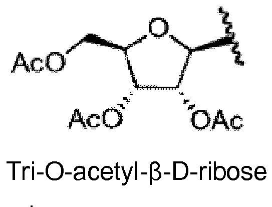 oder 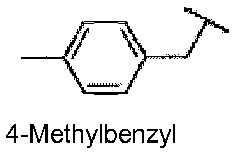 oder 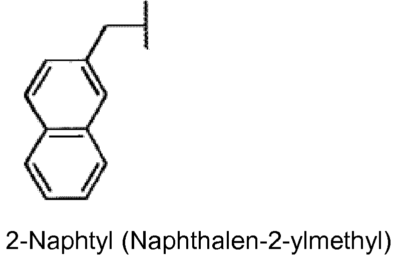 und
- wenn R1 eine β-D-Ribose-Gruppe ist, dann:
- R2 = CONH₂ und R3 = Cl, CO-Furan, CO-Thiophen oder Toluoyl;
oder
- R2 = CO₂Me und R3 = I oder Acetylen;
oder
- R2 = Phenyl und R3 = I;
- wenn R1 eine Tri-O-acetyl-β-D-ribose-Gruppe ist, dann:
- R2 = CO₂Et und R3 = CO-(CH₂)₅-CH₃, CO-Furan, Toluoyle, -C=C-CO₂Et, Thiophene oder Phenyl;
oder
- R2 = Phenyl und R3 = -C≡C-Phenyl;
oder
- R2 = Thiophen und R3 = -C=C-Thiophen;
oder
- R2 = (CH₂)₆CH₃ und R3 = -C≡C-(CH₂)₆CH₃;
oder
- R2 = p-Fluorphenyl und R3 = -C≡C-p-Fluorphenyl;
oder
- R2 = 2-Methoxynaphtalen und R3 = -C≡C-2-Methoxynaphtalen;
- wenn R1 eine 4-Methylbenzyl-Gruppe ist, dann:
- R2 = CO₂Et und R3 = -C≡C-CO₂Et;
oder
- R2 = Phenyl und R3 = -C≡C-Phenyl;
- wenn R1 eine 2-Naphtyl (Naphthalen-2-ylmethyl)-Gruppe ist, dann:
- R2= CO₂Et und R3 = I;
oder
- R2 = CO₂Et und R3 = -C≡C-CO₂Et;
oder R2 = Phenyl und R3 = -C≡C-phenyl.

5. Verbindungen nach Anspruch 4, wobei die Verbindungen ausgewählt sind aus:
- 1'-(4-Ethoxycarbonyl-5-iodo-[1,2,3]-triazol-1-yl)-2',3',5'-tri-O-acetyl-β-D-ribofuranose;
- 1'-(4-Carbamoyl-5-iodo-[1,2,3]-triazol-1-yl)-β-D-ribofuranose;
- 1'-(4-Methoxycarbonyl-5-ethynyl-[1,2,3]-triazol-1-yl)-β-D-ribofuranose;
- 1-(Naphtyl-2-methyl)-4-ethoxycarbonyl-5-iodo-1,2,3-triazol;
- 1-(Naphtyl-2-methyl)-4-ethoxycarbonyl-5-ethylpropiolat-1,2,3-triazol;
- 1'-(4-Ethoxycarbonyl-5-ethylpropiolat-[1,2,3]-triazol-1-yl)-2',3',5'-tri-O-acetyl-β-D-ribofuranose;
- 1'-(4-Ethoxycarbonyl-5-(2-thienyl)-[1,2,3]-triazol-1-yl)-2',3',5'-tri-O-acetyl-β-D-ribofuranose;
- 1'-(4-Ethoxycarbonyl-5-phenyl-[1,2,3]-triazol-1-yl)-2',3',5'-tri-O-acetyl-β-D-Ribofuranose;
- 1-(4-Methylbenzyl)-4-ethoxycarbonyl-5-ethylpropiolat-1,2,3-triazol;
- 1'-(4-Heptyl-5-(non-1-yn-1-yl)-[1,2,3]-triazol-1-yl).2',3',5'-tri-O-acetyl-β-D-ribonfuranose;
- 1'-(4-Ethoxycarbonyl-5-ethylpropiolat-[1,2,3]-triazol-1-yl)-2',3',5'-tri-O-benzoyl-β-L-ribofuranose;
- 2'-Desoxy-1'-(4-ethoxycarbonyl-5-ethylpropiolat-[1,2,3]-triazol-1-yl)-3',5'- di-O-(p-toluoyl)- α-D-ribofuranose
- 1'-(4-Ethoxycarbonyl-5-ethylpropiolat-[1,2,3]-triazol-1-yl)-2',3',4',6'-tetra-O-acetyl-β-D-glucopyranose;
- 1'-(4-Ethoxycarbonyl-5-ethylpropiolat-[1,2,3]-triazol-1-yl)-2',3'-O-isopropyliden-β-D-ribofuranose;
- 1'-(4-Ethoxycarbonyl-5-ethylpropiolat-[1,2,3]-triazol-1-yl)-2',3'-O-isopropyliden-5'-O-acetyl-β-D-ribofuranose;
- 1'-(4-Ethoxycarbonyl-5-(2-thienyl)-[1,2,3]-triazol-1-yl)-2',3'-O-isopropyliden-β-D-ribofuranose, und
- 1'-(4-Ethoxycarbonyl-5-(2-thienyl)-[1,2,3]-triazol-1-yl)-2',3'-O-isopropyliden-5'-O-acetyl-β-D-ribofuranose.

6. Verbindungen nach Anspruch 5, wobei die Verbindungen ausgewählt sind aus:
- 1'-(4-Ethoxycarbonyl-5-(2-thienyl)-[1,2,3]-triazol-1-yl)-2',3',5'-tri-O-acetyl-β-D-ribofuranose;
- 1'-(4-Ethoxycarbonyl-5-ethylpropiolat-[1,2,3]-triazol-1-yl)-2',3',5'-tri-O-acetyl-β-D-ribofuranose;
- 1'-(4-Ethoxycarbonyl-5-ethylpropiolat-[1,2,3]-triazol-1-yl)-2',3'-O-isopropyliden-β-D-ribofuranose,
- 1'-(4-Ethoxycarbonyl-5-ethylpropiolat-[1,2,3]-triazol-1-yl)-2',3'-O-isopropyliden-5'-O-acetyl-β-D-ribofuranose; und
- 1-(4-Methylbenzyl)-4-ethoxycarbonyl-5-ethylpropiolat-1,2,3-triazol.

7. Verbindungen nach Anspruch 5, wobei die Verbindungen ausgewählt sind aus:
- 1'-(4-Ethoxycarbonyl-5-(2-thienyl)-[1,2,3]-triazol-1-yl)-2',3',5'-tri-O-acetyl-β-D-ribofuranose;
- 1'-(4-Ethoxycarbonyl-5-phenyl-[1,2,3]-triazol-1-yl)-2',3',5'-tri-O-acetyl-β-D-ribofuranose;
- 1-(4-Methylbenzyl)-4-ethoxycarbonyl-5-ethylpropiolat-1,2,3-triazol;
- 1'-(4-Ethoxycarbonyl-5-ethylpropiolat-[1,2,3]-triazol-1-yl)-2',3',4',6'-tetra-O-acetyl-β-D-glucopyranose, und
- 1'-(4-Ethoxycarbonyl-5-(2-thienyl)-[1,2,3]-triazol-1-yl)-2',3'-O-isopropyliden-β-D-ribofuranose.

8. Verbindungen nach einem der Ansprüche 1 bis 7 für ihre Verwendung bei der Behandlung von Stoffwechselerkrankungen.

9. Verbindungen nach einem der Ansprüche 1 bis 7 für ihre Verwendung bei der Behandlung von Krebs.

10. Verbindungen nach Anspruch 9 für ihre Verwendung bei der Behandlung von festen Tumoren.

11. Verbindungen nach Anspruch 9 für ihre Verwendung bei der Behandlung von bösartigen Blutkrankheiten.

12. Verbindungen nach einem der Ansprüche 1 bis 7 für ihre Verwendung bei der Behandlung der chronischen myeloischen Leukämien (LMC).

13. Verbindungen nach Anspruch 9 für ihre Verwendung bei der Behandlung von Krebserkrankungen, die gegen Tyrosinkinase-Inhibitoren resistent sind.

14. Verbindungen nach Anspruch 12 für ihre Verwendung bei der Behandlung der chronischen myeloischen Leukämien (LMC), die gegen Tyrosinkinase-Inhibitoren resistent sind.

15. Pharmazeutisches Produkt, enthaltend eine Verbindung nach einem der Ansprüche 1 bis 7 und mindestens einen zweiten Wirkstoff als Kombinationsprodukt für eine gleichzeitige, getrennte oder zeitlich gestaffelte Verabreichung für ihre Verwendung bei der Behandlung von Krebs.

16. Pharmazeutische Zusammensetzung, umfassend eine Verbindung nach einem der Ansprüche 1 bis 7 und einen pharmazeutisch verträglichen Träger.

## Claims

1. Compounds of the general formula : 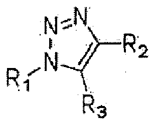 wherein:
- R₁ is selected among
• a cyclic pentose group in the furan form with the free OH groups or optionally substituted by one or several mono-, bi- or tri-phosphate group(s), acetyl group(s), isopropylidene group(s), benzoyl group(s) or para-toluoyle group(s),
• a hexose group in the pyran form with the free OH groups or optionally substituted by one or several mono-, bi- or tri-phosphate group(s) or acetyl group(s),
• a naphthyl group, optionally substituted by one or several substituted alkyl or amino group(s) having from 1 to 4 carbon atom(s),
• a benzyl group, optionally substituted by one or several substituted alkyl or amino group(s) having from 1 to 4 carbon atom(s),
• phenyl, biphenyl, heteroaryl groups ;
- R₂ is selected among
• an amide group -CONH₂, -CONHMe, -CONHEt, -CON(Me)₂,-CON(Et)₂,
• an acid or ester group -CO₂H, CO₂Me, CO₂Et, a cyano or amidine group -CN, -C(NH₂)NH, -C(NHMe)NH, -C(NHEt)NH,
• a phenyl group optionally substituted by a halogen selected among Cl, Br, I an F,
• a thiophene group,
• a straight or branched carbon chain having from 3 to 10 carbon atoms, or
• a methoxynaphthalene group ; and
- R₃ is selected among
• a halogen group,
• a furan or -CO-furan group,
• a thiophene or -CO-thiophene or -C=C-thiophene group,
• a toluoyl group,
• an acetylene group,
• a -CO-(CH₂)ₙ-CH₃ group, wherein n is comprised between 2 and 9,
• a phenyl or -C≡C-phenyl group, optionally substituted by a halogen,
• a -C≡C-CO₂Me, -C≡C-CO₂Et, -C≡C-CONH₂group,
• a -C≡C-(CH₂)₆CH₃ group, or
• a -C≡C-2-methoxynaphthalene group ;
their racemates, enantiomers, diastereoisomers and mixtures thereof, their tautomers and pharmaceutically acceptable salts thereof,
for their use as a medicine.

2. The compounds according to claim 1, wherein :
- R₁ is selected among 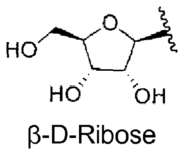 or 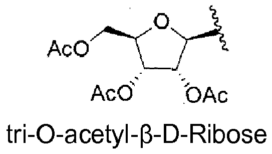 or 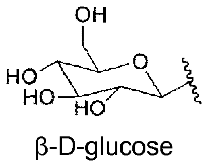 or 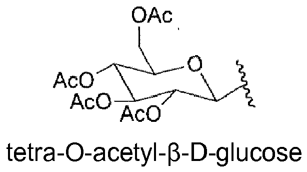 or 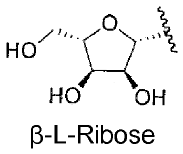 or 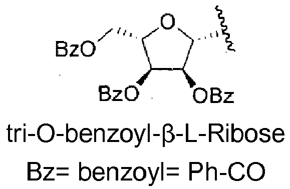 or 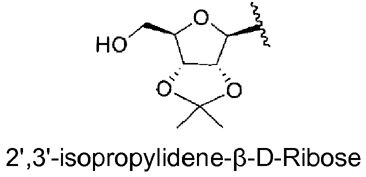 or 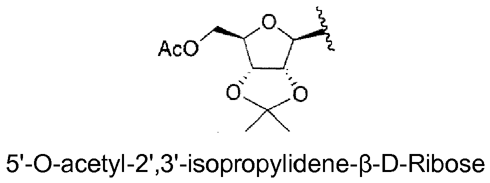 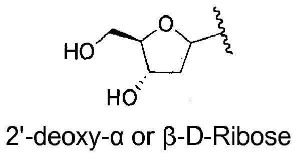 or 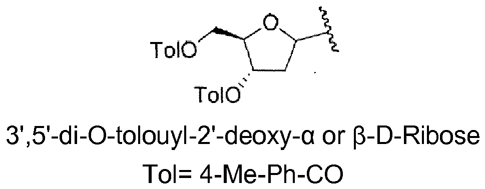 or 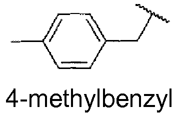 or 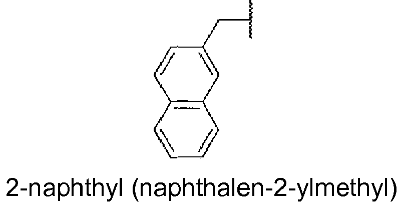
- R₂ is selected among -CONH₂, -CO₂Me, -CO₂Et, phenyl, thiophene,-(CH₂)₆CH₃, p-fluoro-phenyl or 2-methoxynaphthalene ; and
- R₃ is selected among I, Cl, furan, CO-furan, CO-thiophene, acetylene, CO-(CH₂)₅-CH₃, toluoyle, -C≡C-CO₂Et, thiophene, phenyl, -C=C-phenyl, -C=C-thiophene, -C≡C-(CH₂)₆CH₃, -C≡C-(p-fluoro-phenyl), or -C≡C-2-methoxynaphthalene.

3. The compounds according to claim 2, wherein R₁ is 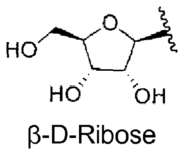 or 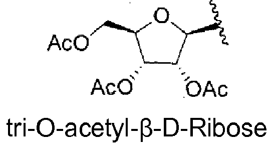 or 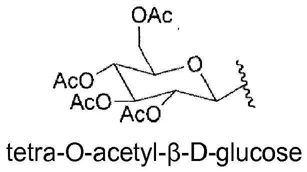 or 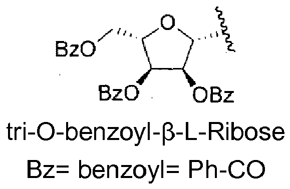 or 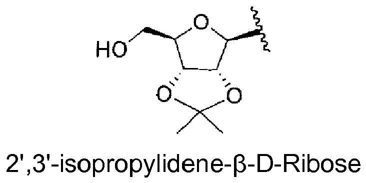 or 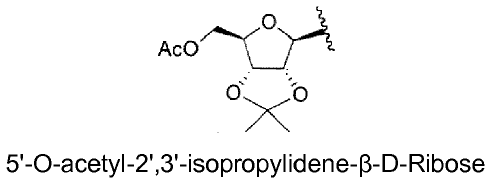 or 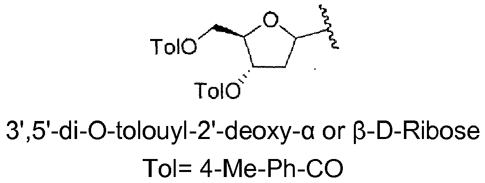 or 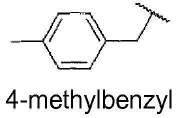 or 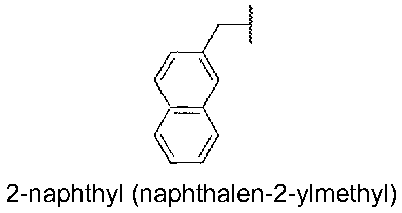 and
- when R₁ is a β-D-ribose group, then :
- R₂= CONH₂ and R₃= I, Cl, CO-furan, CO-thiophene, toluoyle or acetylene ;
or
- R₂= CO₂Me and R₃= I, furan or acetylene ;
or
- R₂= phenyl and R₃= I ;
- when R₁ is a tri-O-acetyl-β-D-ribose group, then :
- R₂= CO₂Et and R₃= Cl, CO-(CH₂)₅-CH₃, CO-furan, toluoyle, -C=C-CO₂Et, thiophene or phenyl ;
or
- R₂= phenyl and R₃= -C≡C-phenyl ;
or
- R₂= thiophene and R₃= -C≡C-thiophene ;
or
- R₂= (CH₂)₆CH₃ and R₃= -C≡C-(CH₂)₆CH₃ ;
or
- R₂= p-fluoro-phenyl and R₃= -C≡C-p-fluoro-phenyl ;
or
- R₂= 2-methoxynaphthalene and
R₃= -C=C-2-methoxynaphthalene ;
- when R₁ is a tetra-O-acetyl-β-D-glucose group, then R₂= CO₂Et and R₃= I or -C≡C-CO₂Et ;
- when R₁ is a tri-O-benzoyl-β-L-ribose group, then R₂= CO₂Et and R₃=-C≡C-CO₂Et ;
- when R₁ is a 2',3'-isopropylidene-β-D-ribose group, then R₂= CO₂Et and R₃= -C≡C-CO₂Et or thiophene ;
- when R₁= 5'-O-acetyl-2',3'-isopropylidene-β-D-ribose, then R₂= CO₂Et and R₃= -C≡C-CO₂Et or thiophene ;
- when R₁ is a 3',5'-di-O-tolouyl-2'-deoxy-β-D-ribose group, then R₂= CO₂Et and R₃= -C≡C-CO₂Et ;
- when R₁ is a 4-methylbenzyl group, then :
- R₂= CO₂Et and R₃= -C≡C-CO₂Et ;
or
- R₂= phenyl and R₃= -C≡C-phenyl ;
- when R₁ is a 2-naphthyl (naphthalen-2-ylmethyl) group, then :
- R₂= CO₂Et and R₃= I ; ;
or
- R₂= CO₂Et and R₃= -C≡C-CO₂Et ;
or
- R₂= phenyl and R₃= -C≡C-phenyl.

4. The compounds according to claim 3, wherein R₁ is 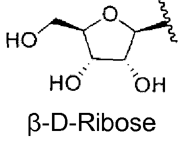 or 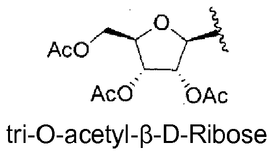 or 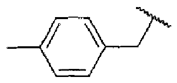 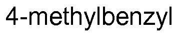 or 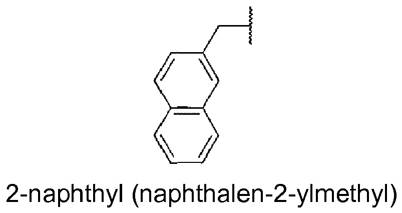 and
- when R₁ is a β-D-ribose group, then :
- R₂= CONH₂ and R₃= Cl, CO-furan, CO-thiophene or toluoyle ;
or
- R₂= CO₂Me and R₃= I or acetylene ;
or
- R₂= phenyl and R₃= I ; ;
- when R₁ is a tri-O-acetyl-β-D-ribose group, then :
- R₂= CO₂Et and R₃= CO-(CH₂)₅-CH₃, CO-furan, toluoyle, -C≡C-CO₂Et, thiophene or phenyl;
or
- R₂= phenyl and R₃= -C≡C-phenyl ;
or
- R₂= thiophene and R₃= -C≡C-thiophene ;
or
- R₂= (CH₂)₆CH₃ and R₃= -C≡C-(CH₂)₆CH₃ ;
or
- R₂= p-fluoro-phenyl and R₃= -C=C-p-fluoro-phenyl ;
or
- R₂= 2-methoxynaphthalene and
R₃= -C=C-2-methoxynaphthalene ;
- when R₁ is a 4-methylbenzyl group, then :
- R₂= CO₂Et and R₃= -C≡C-CO₂Et ;
or
- R₂= phenyl and R₃= -C≡C-phenyl ;
- when R₁ is a 2-naphthyl (naphthalen-2-ylmethyl) group, then :
- R₂= CO₂Et and R₃= I;
or
- R₂= CO₂Et and R₃= -C≡C-CO₂Et ;
or
- R₂= phenyl and R₃= -C≡C-phenyl.

5. The compounds according to claim 4, said compounds being selected among :
- 1'-(4-ethoxycarbonyl-5-iodo-[1,2,3]-triazol-1-yl)-2',3',5'-tri-O-acetyl-β-D-ribofuranose;
- 1'-(4-carbamoyl-5-iodo-[1,2,3]-triazol-1-yl)-β-D-ribofuranose;
- 1'-(4-methoxycarbonyl-5-ethynyl-[1,2,3]-triazol-1-yl)-β-D-ribofuranose;
- 1-(naphthyl-2-methyl)-4-ethoxycarbonyl-5-iodo-1,2,3-triazole;
- 1-(naphthyl-2-methyl)-4-ethoxycarbonyl-5-ethylpropiolate-1,2,3-triazole;
- 1'-(4-ethoxycarbonyl-5-ethylpropiolate-[1,2,3]-triazol-1-yl)-2',3',5'-tri-O-acetyl-β-D-ribofuranose;
- 1'-(4-ethoxycarbonyl-5-(2-thienyl)-[1,2,3]-triazol-1-yl)-2',3',5'-tri-O-acetyl-β-D-ribofuranose;
- 1'-(4-ethoxycarbonyl-5-phenyl-[1,2,3]-triazol-1-yl)-2',3,5'-tri-O-acetyl-β-D-ribofuranose;
- 1-(4-methylbenzyl)-4-ethoxycarbonyl-5-ethylpropiolate-1,2,3-triazole;
- 1'-(4-heptyl-5-(non-1-yn-1-yl)-[1,2,3]-triazol-1-yl)-2',3',5'-tri-O-acetyl-β-D-ribofuranose;
- 1'-(4-ethoxycarbonyl-5-ethylpropiolate-[1,2,3]-triazol-1-yl)-2',3',5'-tri-O-benzoyl-β-L-ribofuranose;
- 2'-desoxy-1'-(4-ethoxycarbonyl-5-ethylpropiolate-[1,2,3]-triazol-1-yl)-3',5'-di-O-(p-toluoyl)-α-D-ribofuranose
- l'-(4-ethoxycarbonyl-5-ethylpropiolate-[1,2,3]-triazol-1-yl)-2',3',4',6'-tetra-O-acetyl-β-D-glucopyranose;
- 1'-(4-ethoxycarbonyl-5-ethylpropiolate-[1,2,3]-triazol-1-yl)-2',3'-O-isopropylidene-β-D-ribofuranose;
- 1'-(4-ethoxycarbonyl-5-ethylpropiolate-[1,2,3]-triazol-1-yl)-2',3'-O-isopropylidene-5'-O-acetyl-β-D-ribofuranose;
- 1'-(4-ethoxycarbonyl-5-(2-thienyl)-[1,2,3]-triazol-1-yl)-2',3'-O-isopropylidene-β-D-ribofuranose; and
- 1'-(4-ethoxycarbonyl-5-(2-thienyl)-[1,2,3]-triazol-1-yl)-2',3'-O-isopropylidene-5'-O-acetyl-β-D-ribofuranose.

6. The compounds according to claim 5, said compounds being selected among :
- 1'-(4-ethoxycarbonyl-5-(2-thienyl)-[1,2,3]-triazol-1-yl)-2',3',5'-tri-O-acetyl-β-D-ribofuranose;
- 1'-(4-ethoxycarbonyl-5-ethylpropiolate-[1,2,3]-triazol-1-yl)-2',3',5'-tri-O-acetyl-β-D-ribofuranose;
- 1'-(4-ethoxycarbonyl-5-ethylpropiolate-[1,2,3]-triazol-1-yl)-2',3'-O-isopropylidene-β-D-ribofuranose;
- 1'-(4-ethoxycarbonyl-5-ethylpropiolate-[1,2,3]-triazol-1-yl)-2',3'-O-isopropylidene-5'-O-acetyl-β-D-ribofuranose; and
- 1-(4-methylbenzyl)-4-ethoxycarbonyl-5-ethylpropiolate-1,2,3-triazole.

7. The compounds according to claim 5, said compounds being selected among :
- 1'-(4-ethoxycarbonyl-5-(2-thienyl)-[1,2,3]-triazol-1-yl)-2',3',5'-tri-O-acetyl-β-D-ribofuranose;
- 1'-(4-ethoxycarbonyl-5-phenyl-[1,2,3]-triazol-1-yl)-2',3',5'-tri-O-acetyl-β-D-ribofuranose;
- 1-(4-methylbenzyl)-4-ethoxycarbonyl-5-ethylpropiolate-1,2,3-triazole;
- 1'-(4-ethoxycarbonyl-5-ethylpropiolate-[1,2,3]-triazol-1-yl)-2',3',4',6'-tetra-O-acetyl-β-D-glucopyranose; and
- 1'-(4-ethoxycarbonyl-5-(2-thienyl)-[1,2,3]-triazol-1-yl)-2',3'-O-isopropylidene-β-D-ribofuranose.

8. The compounds according to any of claims 1 to 7, for their use in the treatment of metabolic diseases.

9. The compounds according to any of claims 1 to 7, for their use in the treatment of cancer.

10. The compounds according to claim 9, for their use in the treatment of solid tumors.

11. The compounds according to claim 9, for their use in the treatment of hematological malignancies.

12. The compounds according to any of claims 1 to 7, for their use in the treatment of chronic myelogenous leukemia (CML).

13. The compounds according to claim 9, for their use in the treatment of cancers resistant to tyrosine kinase inhibitors.

14. The compounds according to claim 12, for their use in the treatment of chronic myelogenous leukemias (CML) resistant to tyrosine kinase inhibitors.

15. A pharmaceutical product containing a compound according to any of claims 1 to 7 and at least a second one active as a combination product for simultaneous, separate or spread over time administration, for its use in the treatment of cancer.

16. A pharmaceutical composition comprising a compound according to any of claims 1 to 7 and a pharmaceutically acceptable carrier.
